# EUROPÄISCHE PATENTSCHRIFT

(11) **EP 1 780 541 B1**
(45) Veröffentlichungstag und Bekanntmachung des Hinweises auf die Patenterteilung: **15.10.2008**
(21) Anmeldenummer: 05023219.8
(22) Anmeldetag: 25.10.2005
(51) Int. Cl.: G01N 33/487, G01N 33/50

(54) **Analysegerät zur Analyse einer Probe auf einem Testelement**
Analysis device for analysing a sample on a test element
Appareil d'analyse destiné à l'analyse d'un échantillon sur un élément de test

(43) Veröffentlichungstag der Anmeldung: 02.05.2007
(73) Patentinhaber: F.HOFFMANN-LA ROCHE AG, 4070 Basel (CH); Roche Diagnostics GmbH, 68305 Mannheim (DE)
(72) Erfinder: Riebel, Stefan, 67069 Ludwigshafen-Edigheim (DE); Wieder, Herbert, 68259 Mannheim (DE); Bainczyk, Gregor, 68199 Mannheim (DE); Augstein, Manfred, 68305 Mannheim (DE); Kube, Oliver, 67547 Worms (DE); Meinecke, Dieter, 68309 Mannheim (DE); Thoes, Bruno, 66287 Quierschied (DE); Grosser, Albert, 58511 Lüdenscheid (DE)
(74) Vertreter: Isenbruck, Günter

(56) Entgegenhaltungen:
- EP-A- 0 732 590
- EP-A- 1 079 379
- EP-A- 1 383 360
- EP-A- 1 457 913
- WO-A-97/02487
- DE-A1- 19 831 394
- US-A- 5 424 035
- US-A1- 2002 057 993
- US-A1- 2002 076 349
- US-A1- 2004 178 216
- US-A1- 2005 143 675
- FRANK PÖHLAU: "Räumliche Schaltungsträger- Rationalisieren durch Integration" SIEMENS- WEBZINE, [Online] Nr. 1/1997, 12. Februar 2001 (2001-02-12), Seiten 1-6, XP002374386 Gefunden im Internet: URL:w4.siemens.de/ful/en/archiv/zeitschrif t/heft1_97/artikel10/index.htlm> [gefunden am 2006-03-26]
- PENTON MEDIA: "Molded Interconnect Devices reshape electromechanical design" ELECTRONIC DESIGN, [Online] 5. September 2000 (2000-09-05), Seiten 1-7, XP002374387 Gefunden im Internet: URL:www.elecdesign.com/Articles/print.cfm? ArticleID=4693> [gefunden am 2006-03-24]
- DAVID C. FRISCH: "Circuitry in Three Dimensions: Multifunctional Molded Plastic Packages" IEEE TRANSACTIONS ON INDUSTRY APPLICATIONS, Bd. 27, Nr. 3, Juni 1991 (1991-06), Seiten 442-446, XP002374388
- FELDMANN K ET AL: "New Requirements And Solutions For Product Data Processing Of Three-dimensional Molded Interconnection Devices" 1992 IEEE INT. ELECTRONICS MANUFACTURING TECHNOLOGY SYMP., 28. September 1992 (1992-09-28), Seiten 94-99, XP010259394
- SPANIER G ET AL: "Biocompatible assembling and packaging technology demonstrated by the integration of a micro sensor on a micro blood pump" PROCEEDINGS OF IEEE SENSORS 2003. 2ND. IEEE INTERNATIONAL CONFERENCE ON SENSORS. TORONTO, CANADA, OCT. 22 - 24, 2003, IEEE INTERNATIONAL CONFERENCE ON SENSORS, NEW YORK, NY : IEEE, US, Bd. VOL. 2 OF 2. CONF. 2, 22. Oktober 2003 (2003-10-22), Seiten 991-995Vol2, XP010691057 ISBN: 0-7803-8133-5
- ERIK JUNG ET AL: "Packaging of an electronic-microfluidic hybrid sensor" 2003 PROCEEDINGS 53RD. ELECTRONIC COMPONENTS AND TECHNOLOGY CONFERENCE. (ECTC). NEW ORLEANS, LA, MAY 27 - 30, 2003, PROCEEDINGS OF THE ELECTRONIC COMPONENTS AND TECHNOLOGY CONFERENCE, NEW YORK, NY : IEEE, US, Bd. CONF. 53, 27. Mai 2003 (2003-05-27), Seiten 373-376, XP010648344 ISBN: 0-7803-7991-5

## Beschreibung

Die vorliegende Erfindung betrifft ein Analysegerät zur Analyse einer Probe auf einem analytischen Testelement, das mindestens ein elektrisch kontaktierendes Bauteil zur Stromübertragung enthält.

Zur Analyse von Proben, beispielsweise Körperflüssigkeiten wie Blut oder Urin, werden häufig Analysegeräte verwendet, bei denen sich die zu analysierenden Proben auf einem Testelement befinden und in einem Testfeld gegebenenfalls mit einer oder mehreren Reagenzien auf dem Testelement reagieren, bevor sie analysiert werden. Die optische, insbesondere photometrische, und die elektrochemische Auswertung von Testelementen stellen die gebräuchlichsten Verfahren zur schnellen Bestimmung der Konzentration von Analyten in Proben dar. Analysesysteme mit Testelementen zur Probenanalyse werden allgemein im Bereich der Analytik, der Umweltanalytik und vor allem im Bereich der medizinischen Diagnostik eingesetzt, Insbesondere im Bereich der Blutglukosediagnostik aus Kapillarblut besitzen Testelemente, die photometrisch oder elektrochemisch ausgewertet werden, einen großen Stellenwert.

Es gibt verschiedene Formen von Testelementen. Bekannt sind zum Beispiel im Wesentlichen quadratische Plättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Im Stand der Technik sind Testelemente umfassend beschrieben, beispielsweise in den Dokumenten DE-A 197 53 847, EP-A 0 821 233, EP-A 0 821 234 oder WO 97/02487. Die vorliegende Erfindung bezieht sich auf Testelemente beliebiger Form, insbesondere auf streifenförmige Testelemente.

Zur analytischen Untersuchung einer Probe auf einem Testelement sind im Stand der Technik Testelement-Analysesysteme bekannt, die eine Testelement-Aufnahme zum Positionieren des Testelements in einer Messposition und eine Mess- und Auswerteeinrichtung zur Durchführung einer Messung und Ermittlung eines hierauf resultierenden Analyseresultats enthalten.

WO 00/19185 A1 bezieht sich auf eine Vorrichtung zur photometrischen Auswertung von Testelementen, beinhaltend
- eine Beleuchtungseinheit mit mindestens einer ersten und einer zweiten Lichtquelle,
- eine Halterung zur Aufnahme eines Testelements mit einer Nachweiszone in der Weise, dass die Nachweiszone gegenüber der Beleuchtungseinheit positioniert wird,
- eine Detektionseinheit mit mindestens einem Detektor, der von der Nachweiszone reflektiertes oder durch die Nachweiszone transmittiertes Licht detektiert,
- eine Steuerungseinheit, die die beiden Lichtquellen aktiviert und das von der Detektionseinheit generierte Signal als Detektionssignal aufnimmt und
- eine Auswerteeinheit, die die Detektionssignale auswertet, um die in der Probe enthaltene Analytkonzentration zu ermitteln.

Weitere Analysegeräte sind z. B. aus EP 0 618 443 A1 oder WO 01/48461 A1 bekannt.

Das von Roche Diagnostics entwickelte Blutzuckeranalysegerät Accu-Chek® Compact Plus misst den Blutzuckerwert nach dem photometrischen Messprinzip. Ein Farbumschlag in einem Testfeld auf einem Testelement, das zuvor mit Blut eines Patienten benetzt wurde, wird dabei von einem optischen Messmodul detektiert und im Gerät elektronisch in einen dem Blutzucker proportionalen Wert umgewandelt. Über einen Einschalttaster wird der Messvorgang gestartet. Daraufhin dreht ein Motor in einem Motormodul die als Vorratsbehältnis dienende Trommel mit den Testelementen um eine Kammer des Vorratsbehältnisses weiter und ein zweiter Motor schiebt mit Hilfe einer Schubstange ein Testelement aus, so dass es vom Anwender außerhalb des Gerätes mit Blut benetzt werden kann. Dabei verbleibt das Testelement so weit im Analysegerät, dass das Testfeld mit der Indikatorchemie im Messmodul über der Messoptik positioniert wird. Diese besteht aus zwei Dioden, einer Photozelle und einer Linse. Die Änderung der diffusen Reflektion wird von der Photozelle in einen Signalstrom umgewandelt, der über eine elektronische Schaltung auf einer Leiterplatte verarbeitet und in einem LC-Display als Blutzuckerwert angezeigt wird. Eine erneute Betätigung des Einschalttasters beendet den Messvorgang, schiebt das Testelement aus und schaltet das Analysegerät ab. Versorgt wird die Mess- und Antriebselektronik über zwei Batterien mit einer Gesamtspannung von ca. 3 V. Im Gegensatz zu vergleichbaren Analysegeräten, in die z. B. Testelemente von außen zugeführt und einzelne Zwischenzustände manuell betätigt werden müssen, weist dieses Gerät eine erhöhte Funktionsintegration auf. 17 einzelne Testelemente werden in dem trommelförmigen Vorratsbehältnis gelagert, dessen Spezifikation mittels eines Barcodereaders im Analysegerät automatisch erkannt wird. Ein Wechsel des trommelförmigen Testelementmagazins wird über einen Schalter am Gehäuseoberteil nach dem Öffnen und Schließen des Testelementmagazinaufnahmedeckels detektiert. Die notwendigen Zustände, wie eine Drehung des Testelementmagazins um einen Schritt und verschiedene Haltepositionen während des Testelementvorschubs, werden über Schleif- und dynamisch federnde Schaltkontakte zur Elektronik der Leiterplatte gemeldet, ohne dass eine Bedienfunktion durch den Anwender notwendig ist. Dynamisch federnd bedeutet in diesem Zusammenhang: Kraftbe- und entlastung sowie mehrmalige Verlagerung im Betrieb. Das Herzstück des Analysegeräts zur Ausführung dieser elektromechanischen Funktionen bildet das Motormodul. Es dient unter anderem zur Aufnahme der Antriebsmotoren und der Getriebeplatine, Die Leiterplatte ist mit dem Motormodul verschraubt. Dazu sind alle weiteren Kontakte zwischen diesen beiden Baugruppen als lösbare statische Federkontakte ausgeführt, wie z. B. die Kontakte zur Stromversorgung der Antriebsmotoren und des Messmoduls. Statisch federnd bedeutet in diesem Zusammenhang: einmalige Kraftbelastung und Verlagerung bei Gerätemontage. Aufgrund der zahlreichen in das Gerät integrierten Funktionen, die von der Gerätesoftware gesteuert werden müssen, ist die Leiterplatte vierlagig ausgeführt.

Die im Stand der Technik bekannten Analysegeräte weisen eine Vielzahl elektrisch kontaktierender Bauteile auf. Beispielsweise bestehen bei dem Accu-Chek® Compact Plus von Roche Diagnostics federnde Kontakte, Steckkontakte, Lötkontakte oder Schleifkontakte, die die Baugruppen Leiterplatte, Motormodul, Messmodul, Trommeldeckelschalter, Barcodereader und LCD miteinander verbinden. Im Stand der Technik werden dazu metallische Stanz- und Biegeteile eingesetzt, die an den einzelnen Baugruppen positioniert und montiert werden müssen, wodurch sich eine große Anzahl an einzelnen Bauteilen, ein hoher Montageaufwand und lange Toleranzketten ergeben. Ferner ist die Gestaltungsfreiheit des elektrisch kontaktierenden Bauteils bei dem Einsatz von Stanze und Biegeteilen begrenzt.

Aufgabe der vorliegenden Erfindung ist es daher, die Nachteile des Standes der Technik zu vermeiden und ein Analysegerät zur Analyse einer Probe auf einem Testelement mit elektrisch kontaktierenden Bauteilen bereitzustellen, indem eine zuverlässig Kontaktierung bei, einer verringerten Anzahl zu montierender Einzelteile gewährleistet ist.

Diese Aufgabe wird erfindungsgemäß gelöst durch ein Analysengerät zur Analyse einer Probe auf einem Testelement, enthaltend mindestens ein elektrisch kontaktierendes Bauteil zur Stromübertragung, das zur Herstellung eines elektrischen Kontakts zu mindestens einem weiteren Bauteil geeignet ist. Das elektrisch kontaktierende Bauteil ist dabei ein spritzgegossener Schaltungsträger (MID), insbesondere ein dreidimensionaler spritzgegossener Schaltungsträger.

Spritzgegossene Schaltungsträger (Molded Interconnect Devices - MID) und Verfahren zu deren Herstellung sind im Stand der Technik bekannt, beispielsweise aus DE 197 17 882 A1, WO 00/67982 A1 oder EP 1383 360 A1.

Unter dem Begriff MID-Technologie sind verschiedene Verfahren zusammengefasst, mit denen räumliche elektronische Baugruppen hergestellt werden können. Das Ziel dieser Verfahren ist die Integration eines Schaltungsbildes in ein polymeres (meist thermoplastisches) Trägerbauteil.

Betrachtet man die konventionellen Lösungen zur elektrischen Kontaktierung und elektronischen Schaltungen in Geräten, findet man häufig Federkontakte, die in ein Gehäuse eingestreckt, geklebt oder heißverstemmt sind (z. B. Batteriekontakte in vielen Kleingeräten). Eine andere Variante sind metallische Blattfedern oder Schleifkontakte, die die peripheren elektrischen Bauteile zu einer Leiterplatte kontaktieren (z. B. Schiebeschalter, Taster oder Stellräder). Die elektronische Schaltung, die die Gerätefunktion steuert, ist meist auf einer Leiterplatte verwirklicht. Die Leiterplatte ist mit den erforderlichen Elektronikbauteilen bestückt. In einfachsten Schaltungen werden zur Verdrahtung Kabel angelötet oder über Stecker miteinander verbunden. Alle diese Aufbau- und Verbindungstechniken haben gemeinsam, dass zum Aufbau eines funktionsfähigen Systems mehrere Bauteile automatisch oder in Handarbeit positioniert, gefügt und montiert werden müssen. Dabei kann entweder ein erheblicher Anlagenaufwand im Falle automatisierter Fertigung oder ein erheblicher Zeit- und Personalaufwand im Falle nichtautomatisierter Fertigung entstehen.

Der Einsatz von MID-Technologien zur Herstellung elektrisch kontaktierender Bauteile in einem Analysegerät bietet hingegen die Möglichkeit, diese Nachteile konventioneller Lösungen zu reduzieren oder zu beseitigen; Montageprozesse können verkürzt oder ganz vermieden werden. Die Bauteilanzahl wird verringert und Toleranzketten werden verkürzt. Im Gerät kommen weniger verschiedenartige Materialien zum Einsatz, was die Entsorgung und Recyclierbarkeit vereinfacht Einige MID-Verfahren bieten darüber hinaus Funktionen, die mit konventionellen Lösungen nicht möglich sind.

MID-Verfahren beruhen auf dem selektiven Aufbringen von elektrisch leitfähigen Metallschichten auf Spritzgießkunststoffteilen. Ein solches metallisiertes polymeres Spritzgießteil kann elektrische Funktionen (z. B. die Funktion von Leiterbahnen, Steck- oder Schleifkontakten) und mechanische Funktionen (z. B. die Funktion als Befestigungselement) enthalten.

Die wichtigsten MID-Verfahren, durch die bei dem erfindungsgemäßen Analysegerät der spritzgegossene Schaltungsträger hergestellt werden kann, sind
- das Zweikomponentenspritzgießen,
- das Heißprägen,
- das Folienhinterspritzen und
- die Laserstrukturierung.

Beim Zweikomponentenspritzgießen werden eine metallisierbare und eine nicht-metallisierbare thermoplastische Werkstoffkomponente in zwei Arbeitsschritten aufeinander gespritzt. Beim zweiten Schuss wird dabei das Werkstück im Werkzeug auf den Flächen abgestützt, die während des ersten Schusses ihre endgültige Kontur erhalten haben. Nach dem Spritzgießen wird die Oberfläche des metallisierbaren thermoplastischen Kunststoffs ggf. aktiviert und die gewünschte Metallschichtdicke (z. B. Kupferschichtdicke) chemisch oder galvanisch aufgebracht. Im anschließenden Schritt wird eine Oberflächenveredelung, z. B. Ni-Au, appliziert. Der metallisierbaren Werkstoffkomponente wird z. B. Palladium beigemischt, das als Keim für die Metallisierung dient. Die Palladiumkeime dienen z. B. als Zerfallszentren für stabilisierte Nickel- oder Kupferlösungen. Bei anderen Verfahren kommen inhärent metallisierbare Kunststoffe zum Einsatz. Bei den Metallisierungsverfahren wird zwischen außenstromloser chemischer Metallisierung (ohne Stromquelle) und galvanischer Metallabscheidung (bei anliegender Stromquelle) unterschieden,

Mögliche für das Zweikomponentenspritzgießverfahren einsetzbare metallisierbare Komponenten sind z. B. PES (Polyethersulfon), PEI (Polyetherimid), LCP (Flüssigkristallpolymer), PA (Polyamid), PPA (Polyphthalamid) oder ABS (Acrylnitril-Butadien-Styrol). Als nicht-metallisierbare Komponenten können z. B. ABS + PSU (Acrylnitril-Butadien-Styrol + Polysulfon), PPA (Polyphthalamid), PBT (Polybutylenterephthalat), PPS (Polyphenylensulfid), PES (polyethersulfon), PC (Polycarbonat) oder PA (Polyamid) eingesetzt werden.

Beim Heißprägen wird nach dem Spritzgießen in einem Arbeitsschritt durch Ausstanzen und Aufprägen einer Metallfolie (z. B. Kupferfolie) auf das Kunststoffsubstrat die metallische Struktur (z. B. die Leiterbahnen) aufgebracht. Bei der Heißprägetechnik sind Durchkontaktierungen durch Füllen von Bohrungen mit Leitpaste zu realisieren. Beim Heißprägen ist außer dem Einkomponentenspritzgießen des Substrates noch die Folienherstellung notwendig. Heißprägefolien für MID-Anwendungen sind in der Regel durch einen Dreischichtaufbau aus einer leitfähigen Kupferfolie, einer Haftschicht auf der Unterseite und einer als Oxidationsschicht sowie zur besseren Löt- und Kontaktierbarkeit dienenden Oberflächenmetallisierung gekennzeichnet. Die Kupferfolien werden elektrolytisch auf einer Kupfersulfatlösung direkt auf einer rotierenden Titanwalze abgeschieden. Die im Prägeprozess zum Ausscheren der metallischen Strukturen (Leiterbahnen) erforderliche geringe Scherfestigkeit der Kupferfolie wird durch eine spezielle Verfahrenstechnik bei der Folienherstellung erreicht, die zu einem orientierten Kristallwachstum senkrecht zur Folienoberfläche führt Vor Anwendungen unterschiedlicher Strombelastung werden Kupferfolien mit Schichtdicken 12, 18, 35 und 100 µm hergestellt, wobei die in der Leiterplattentechnik häufig verwendete Schichtdicke von 35 µm typisch ist. Die Haftfestigkeit der Folie auf dem Substrat wird entweder durch eine Klebstoffschicht auf der Folienunterseite oder einer Strukturierung der Folienunterseite erreicht. Mögliche für das Heißprägeverfahren einsetzbare Werkstoffe sind z. B. ABS (Acrylnitril-Butadien-Styrol), PA (Polyamid), PBT (Polybutylenterephthalat), PC + ABS (Polycarbonat + Acrylnitril-Butadien-Styrol) und PPS (Polyphenylensulfid).

Beim Folienhinterspritzen erfolgt zunächst die Strukturierung des gewünschten Schaltbildes auf einer Kunststofffolie. Die Folie kann z. B. mit subtraktiver Flex-Leiterplattentechnik oder additiv mittels Primertechnologie oder Heißprägeverfahren strukturiert werden. Bei der Flex-Leiterplattentechnik wird die Polymerfolie (z. B. aus Polyamid) ganzflächig metallisiert und anschließend in Subtraktivtechnik über Ätzprozesse strukturiert. Bei der Primeitechnologie wird über Druckverfahren ein metallisierbarer Haftvermittler auf die Kunststofffolie aufgetragen.

Nach der Strukturierung kann die Folie umgeformt und anschließend hinterspritzt werden. Die Metallisierung des Kunststoffteils kann vor oder nach dem Hinterspritzen erfolgen.

Die hinterspritzte Kunststofffolie kann z. B. PEI (Polyetherimid), PC (Polycarbonat) oder PC/PBT (Polycarbonat/Polybutylenterephthalat) enthalten. Einsetzbare Materialien zum Hinterspritzen der Folie sind z. B. PEI (Polyetherimid), PC (Polycarbonat), PBT (Polybutylenterephthalat), PET (Polyethylenterephthalat) oder PEN (polyethylennaphtenat).

Ein weiteres mögliches Verfahren zur Herstellung des (dreidimensionalen) spritzgegossenen Schaltungsträgers für das erfindungsgemäße Analysegerät ist die Laserstrukturierung.

Bei der subtraktiven Laserstrukturierung wird das spritzgegossene Werkstück vor der Strukturierung der Leiterbahnen ganzflächig erst nasschemisch und dann elektrolytisch bis zur gewünschten Endschichtdicke verkupfert. Auf die Kupferschicht wird ein Ätzresist aufgebracht, z. B. ein Photoresist, bei dem mittels UV-Strahlung eingebrachte Energie eine chemische Reaktion auslöst oder ein Galvanoresist, das mittels Laserstrahlen abgetragen wird. Die Struktunerung des Resists erfolgt dann mittels Laserstrahl und in den strukturierten Bereichen wird anschließend das Kupfer weggeätzt. Danach erfolgt eine Oberflächenveredelung.

Bei der additiven Laserstrukturierung werden thermoplastische Werkstoffe derart modifiziert, dass eine metallorganische Komplexverbindung gelöst oder feindispergiert im Werkstoff vorliegt Auf den so dotierten Thermoplasten werden dann die zu realisierenden Leiterbahnen mittels Laser gezielt aktiviert und anschließend in einem chemischen Bad metallisiert. Für diesen Prozess werden in der Regel chemische Kupferelektrolyten verwendet, die typischerweise 5 bis 8 µm dicke Kupferschichten erzeugen. Anschließend kann ein geeignetes Oberflächenfinish aufgebracht werden.

Die Lasezstrukturierung ist z. B. auf PEI (Polyetherimid), PA (Polyamid), LCP (Flüssigkkristallpolymer), ABS (Acrylnitril-Butadien-Styrol), PC (Polycarbonat), PC + ABS (Polycarbonat + Acrylnitril-Butadien-Styrol), PBT (Polybutylenterephthalat), PI (Polyimid) oder PET (Polyethylenterephthalat) anwendbar, wobei der Werkstoff gegebenenfalls dotiert werden muss.

Das erfindungsgemäße Analysegerät kann eine Probe auf einem Testelement z. B. photometrisch und/oder elektrochemisch analysieren.

In dem erfindungsgemäßen Analysegerät liegt mindestens ein elektrisch kontaktierendes Bauteil als dreidimensionaler spritzgegossener Schaltungsträger vor, der nach einem der beschriebenen Verfahren hergestellt worden sein kann. Das elektrisch kontaktierende Bauteil weist mindestens eine federnde Kontaktfahne zur Herstellung eines elektrischen Kontakts zu mindestens einem weiteren Bauteil auf, in form eines Schleifkontakts. Ausgehend von einem solchen elektrischen Kontakt führen auf diesem spritzgegossenen Schaltungsträger (MID-Bauteil) Leiterbahnen zu einem elektrischen Bauelement, z. B. einem Sensor, einem Barcodereader, einem weiteren Kontakt, einem Motor etc. Als federnde Kontakte werden sowohl einfach, als auch mehrfach statisch federnd kontaktierende Kontakte bezeichnet.

Das elektrisch kontaktierende Bauteil weist mindestens eine federnde Kontaktfahne zur Kontaktierung des zweiten Bauteils auf, die einen Kontaktfahnenkörper aus spritzgegossenem Kunststoff und eine metallische Stromleiterstruktur enthält. Als spritzgegossener Kunststoff kommt z.B. PEI (Polyetherimid), PA (polyamid), LCP (Flüssigkkristallpolymer), ABS (Acrylnitril-Butadien-Styrol), PC (Polycarbonat), PC + ABS (Polycarbonat + Acrylnitril-Butadien-Styrol). PET (Polybutylenterephthalat), PI (Polyimid) oder PET (Polyethylenterephthalat) und für die metallische Stromleiterstruktur kommt z.B. Kupfer, Gold oder Nickel zum Einsatz. Die Kontaktfahne kontaktiert in oder an dem Analysengerät ein rotierendes oder ein linear bewegtes weiteres Bauteil des Analysegeräts.

In dem erfindungsgemäßen Analysegerät kann das als spritzgegossener Schaltungsträger ausgeführte kontaktierende Bauteil z. B. eine funktionale Baugruppe des Analysegeräts und das weitere kontaktierte Bauteil eine Leiterplatte des Analysegeräts sein. Funktionale Baugruppen sind dabei z. B. eine Barcodereaderanordnung, ein Gehäuse, eine Transporteinheit für Testelement, ein Motormodul, ein Messmodul, eine Positioniereinrichtung für ein Testelementmagazin, eine einen Sensor enthaltende Testelementmagazinaufnahme oder eine Temperiereinrichtung in einem Messmodul.

Eine Barcodereaderanordnung ist z. B. in dem erfindungsgemäßen Analysegerät enthalten, um mittels des Barcodereaders einen Strichcode an einem Vorratsbehältnis für Testelemente einzulesen, der z. B. Informationen über die darin enthaltenen Testelemente und deren optimale Auswertung enthält Im Stand der Technik ist der Barcodereader üblicherweise an einem Leiterplattenarm befestigt, der in einen Gehäuseabschnitt des Analysegeräts hineinragt, in dem ein Vorratsbehältnis für Testelemente (Testelementmagazin) aufgenommen werden kann. Die elektrische Kontaktierung des Barcodereaders erfolgt dabei über den verlängerten Leiteiplattenarm.

Vorzugsweise enthält das erfindungsgemäße Analysegerät im Unterschied dazu eine Barcodereaderanordnung, die einen Gehäuseabscbuitt des Analysegeräts umfasst, in dem ein Barcodereader angeordnet ist, wobei der Gehäuseabschnitt zu dem Barcodereader verlaufende Leiterbahnen und federnde Kontakte zum Kontaktieren einer Leiterplatte enthält und der Gehäuseabschnitt mit den Leiterbahnen und federnden Kontakten ein spritzgegossener Schaltungsträger, insbesondere ein dreidimensionaler spritzgegossener Schaltungsträger ist. In dieser Ausführungsform kann der verlängerte Leiterplattenarm entfallen. Der Barcodereader ist direkt im Gehäuseinneren mit dem Gehäuseabschnitt (z. B. per Lötkontakt) verbunden. Der Gehäuseabschnitt selbst enthält die notwendigen Leiterbahnen und einen statisch federnden Kontakt zum Kontaktieren der Leiterplatte, um die Stromversorgung des Barcodereaders zu gewährleisten. Somit werden Bauteile und Montageschritte eingespart und es wird innerhalb des Analysegeräts Bauraum gewonnen.

Eine Positioniereinrichtung für ein Testelementmagazin ist z. B. bei einer automatischen Entnahme eines Testelements aus dem Testelementmagazin erforderlich, um einen gezielten Zugriff auf das Testelement zu erlauben. Eine solche Positioniereinrichtung rotiert z. B. ein trommelförmiges Testelementmagazin, das, wie in DE 198 54 316 A1 beschrieben, aufgebaut sein kann. Sobald das Testelementmagazin korrekt positioniert ist, kann daraus ein Testelement durch eine Transporteinheit entnommen und in dem Analysegerät weitertransportiert werden.

Im Stand der Technik ist eine Positioniereinrichtung für ein trommelförmiges Testelementmagazin z. B. so aufgebaut, dass ein als Antriebsrad für das Testelementmagazin vorgesehenes Trommelrad mit dem Testelementmagazin mitrotiert und die Trommeldrehung durch zwei auf eine Getriebeplatine heißverstemmte Federkontakte und einen segmentierten Schleifring auf dem Trommelrad registriert wird (insgesamt fünf zu montierende Einzelbauteile).

Gemäß einer bevorzugten Ausführungsform der vorliegenden Erfindung enthält das erfindungsgemäße Analysegerät eine Positioniereinrichtung für ein Testelementmagazin, die eine Platine zur Stromversorgung und ein durch einen Motor angetriebenes Antriebsrad zum Antreiben des drehbaren Testelementmagazins umfasst, wobei die Platine mit Federkontakten als spritzgegossener Schaltungsträger und das Antriebsrad mit einer elektrisch kontaktierbaren Segmentscheibe als spritzgegossener Schaltungsträger ausgeführt ist. Somit werden die fünf Einzelbauteile durch zwei MID-Bauteile ersetzt. Dabei bietet sich ferner der Vorteil einer flexibleren Bauraumgestaltung in dem erfindungsgemäßen Analysegerät durch die freiere Formgestaltung eines Kunststoffteils und der darauf angeordneten Leiterbahnen in MID-Technologie.

Eine Transporteinheit für Testelemente dient in einem Analysegerät zur Analyse einer Probe auf einem Testelement zum Transport eines Testelements in dem Analysegerät, z. B. aus einem Testelementmagazin, in eine Probenaufgabeposition und in eine Messposition. Eine solche Transporteinheit ist beispielsweise aus DE 199 02 601 A1 bekannt. Diese Offenlegungsschrift betrifft ein Verfahren und eine Vorrichtung zum Entnehmen eines analytischen Verbrauchsmittels, insbesondere eines Testelements, aus einem Vorratsbehältnis mit Kammern, die durch Folien verschlossen sind und aus denen das Verbrauchsmittel durch einen Stößel herausgeschoben wird.

Der Stößel (Schubstange) wird durch einen Motor in axialer Richtung verschoben. Dabei weist die Schubstange an einem Ende eine Führungsbuchse auf, die beim Verschieben der Schubstange in einer Führungseinrichtung geführt wird. Die Führungseinrichtung weist ein als Stanz-Biegeteil ausgeführtes Kontaktbauteil mit drei Kontaktfahnen auf, die zur Positionierung der Führungsbuchse und damit der Schubstange dienen. Dazu werden die Kontaktfahnen, je nach Position der Führungsbuchse, durch diese gegen Kontaktflächen auf einer Leiterplatte gedrückt

Gemäß einer Ausfübrungsform der vorliegenden Erfindung enthält das erfindungsgemäße Analysegerät eine Transporteinheit für Testelemente, die eine Schubstange zum Transport eines Testelements innerhalb des Analysegeräts umfasst, wobei die Schubstange eine Führungsbuchse aufweist, die in einer Führungseinrichtung beim Transport des Testelements führbar ist. Dabei weist die Führungsbuchse federnde Kontaktfahnen zum Kontaktieren einer Leiterplatte auf und die Führungseinrichtung umfasst Schaltelemente, die so angeordnet sind, dass sie die Kontakrtfahnen in bestimmten Positionen der Führungsbuchse in der Führungseinrichtung gegen die Leiterplatte drücken, wobei die Führungsbuchse mit den Kontaktfahnen ein spritzgegossener Schaltungsträger (insbesondere ein dreidimensionaler spritzgegossener Schaltungsträger) ist Daher wird die Schaltfunktion in die Führungsbuchse integriert, deren Kontaktfahnen z. B. über spritzgegossene Schaltnocken an der Führungseinrichtung gegen die Leiterplattenkontakte gedrückt werden. Dadurch werden nicht nur Fertigungs-, Positionierungs- und Montageschritte gegenüber dem Stand der Technik eingespart, es ist auch die Möglichkeit zur Platzeinsparung bei entsprechender Gestaltung der Führungsbuchse gegeben.

Weitgehend automatisierte Analysegeräte zur Analyse einer Probe auf einem Testelement enthalten im Stand der Technik Motormodule, die Motoren umfassen. Diese Motoren sind Elektromotoren und dienen z. B. zum Positionieren eines Testelementmagazins oder zum Antrieb einer Schubstange, die ein Testelement innerhalb des Analysegeräts verschiebt.

Im Stand der Technik wird zur Stromversorgung eines Motors (Trommelmotor oder Schubstangenmotor) in einen Motorhalter ein Kontaktblech eingepresst, das über federnde Kontaktfahnen eine elektrisch leitende Verbindung mit einer Leiterplatte herstellte.

Gemäß einer bevorzugten Ausführungsform der vorliegenden Erfindung enthält das erfindungsgemäße Analysegerät ein Motormodul, das einen Motor und einen Motorhalter umfasst, wobei der Motorhalter Kontaktfahnen zum Kontaktieren einer Leiterplatte aufweist und der Motorhalter mit den Kontaktfahnen ein spritzgegossener Schaltungsträger, insbesondere ein dreidimensionaler spritzgegossener Schaltungsträger ist Die Kontaktfahnen des MID-Teiles können an eine Leiterplatte gelötet werden. Durch die Verwendung des spritzgegossenen Schaltungsträgers können Fertigungs- und Montageschritte eingespart werden. Insbesondere kann der Motorhalter einfacher gestaltet werden, da z. B. angespritzte Dome zur Aufnahme des separaten Kontaktblechs wegfallen können.

Ein Analysegerät zur Analyse einer Probe auf einem Testelement, das zur Bevorratung einer Vielzahl von Testelementen ein Testelementmagazin in einer Testelementmagazinaufnahme aufnehmen kann, kann erfindungsgemäß ferner einen Sensor in der Testelementmagazinaufnahme enthalten, der einen Wechsel des Testelementmagazins detektiert. Bei den Analysegeräten aus dem Stand der Technik wird ein Wechsel des Magazins registriert, sobald ein Öffnen und anschließendes Schließen des Analysegerätdeckels, der die Testelementaufnahme abdeckt, erfolgt, da hierbei ein Verriegelungsschalter auf der Leiterplatte betätigt wird. Wird der Analysegerätdeckel jedoch ohne einen Magazinwechsel geöffnet und geschlossen, so registriert das Gerät einen Magazinwechsel, obwohl dieser nicht stattgefunden hat Dies hat zur Folge, dass das Magazin zur Identifikation durch den Barcodereader und zum Auffinden einer mit einem Testelement gefüllten Kammer gedreht wird, ohne dass dies erforderlich wäre, Ein Sensor zum Erfassen eines tatsächlichen Magazinwechsels ist im Stand der Technik nicht vorhanden, da die Stelle, an der der Magazinwechsel zu detektieren wäre, nicht über die im Analysegerät vorhandene zweidimensionale Leiterplatte erreicht werden kann und eine Kontaktierung z. B. über angelötete, flexible Kabel nicht wirtschaftlich einsetzbar ist.

Gemäß einer Ausführungsform der vorliegenden Erfindung umfasst das erfindungsgemäße Analysegerät eine einen Sensor enthaltende Testelementmagazinaufnahme, die zu dem Sensor verlaufende elektrische Leiterbahnen enthält, wobei die Testelementmagazinaufnahme mit den Leiterbahnen ein spritzgegossener Schaltungsträger, insbesondere ein dreidimensionaler spritzgegossener Schaltungsträger ist. Über elektrische Leiterbahnen kann dann ein Signal zur Leiterplatte weitergeleitet werden, falls der Sensor aktiviert würde, z. B. durch Schließen eines Kontakts beim Entnehmen des Magazins.

Ein Analysegerät zur Analyse einer Probe auf einem Testelement enthält gegebenenfalls eine Temperiereinrichtung, um ein Testelement vor und während der Analyse zu temperieren (z. B. bei einer Messung der Blutgerinnung). Im Stand der Technik sind sowohl geräteseitige Heizer (z. B. in einem Messmodul, in dem eine Messung mit der Probe auf dem Testelement durchgeführt wird), als auch in das Testelement integrierte Heizelemente (z. B. aus DE 103 59 160 A1) bekannt Als geräteseitige Heizer kommen Keramikelemente zum Einsatz, die in die Testelementaufnahme montiert werden oder die beim Herstellprozess der Testelementaufnahme in diese eingearbeitet werden. Bei montierten Heizelementen kommt es jedoch häufig zu Problemen mit der Dichtigkeit. Es ist möglich, dass eine Flüssigkeit ins Gerät eindringt und zu Schäden führt. Weitere Probleme treten auf, wenn es zu einer Verschmutzung durch Probenmaterial kommt Es sind ferner mehrere Montageschritte notwendig, um die fertige Baugruppe zu erhalten. Der Einsatz von Keramikheizern hat allgemein den Nachteil, dass es zu einem Keramikbruch kommen kann und dadurch die Funktionsfähigkeit des Gerätes nicht gewährleistet ist. Der Einsatz von integrierten Keramikheizem stellt außerdem hohe Ansprüche an die eingesetzten Fertigungsverfahren und erhöht deren Komplexität beträchtlich.

Gemäß einer Ausführungsform der vorliegenden Erfindung enthält das erfindungsgemäße Analysegerät eine Testelementaufnahme zum Aufnehmen eines Testelements (z.B. während einer Messung), in der eine Temperiereinrichtung zum Temperieren des Testelements enthalten ist, wobei die Temperiereinrichtung Heizwendeln umfasst und ein spritzgegossener Schaltungsträger, insbesondere ein dreidimensionaler spritzgegossener Schaltungsträger ist. Die als Heizwendeln dienenden Leiterbahnen werden also mittels MID-Technik direkt in den Kunststoff der Testelementaufnahme integriert. Die Integration eines Keramikheizers ist nicht erforderlich. Die Kontakte zur elektrischen Stromversorgung der Heizwendeln können z.B. aus metallischen Stanz- oder Biegeteilen oder ebenfalls durch das MID-Verfahren hergestellt werden. Sie ermöglichen ein Anschließen der Temperiereinrichtung an eine Leiterplatte zur Stromversorgung. Die Vorteile dieser Ausführungsform des erfindungsgemäßen Analysegeräts mit Temperiereinrichtung sind eine Vereinfachung des Herstellprozesses, eine Vermeidung der Probleme durch Keramikbruch, eine Reduzierung der Herstellkosten durch Wegfall der Keramikferfigung eine dichte Testelementaufnahme und dass komplexe und miniaturisierte Formen möglich sind.

Gemäß einer weiteren Ausführungsform der vorliegenden Erfindung enthält das erfindungsgemäße Analysegerät ein Messmodul zum Durchführen einer Messung an einem auf einem Testelement enthaltenen Analyten, das eine Testelementaufnahme zum Aufnehmen des Testelements während der Messung umfasst, in der eine Temperiereinrichtung zum Temperieren des Testelements enthalten ist, wobei die Testelementaufnahme mit der Temperiereinrichtung, die Heizwendeln und einen federnden Kontakt umfasst, ein spritzgegossener Schaltungsträger, insbesondere ein dreidimensionaler spritzgegossener Schaltungsträger ist. Die als Heizwendeln dienenden Leiterbahnen werden also mittels MID-Technik direkt in den Kunststoff der Testelementaufuahme integriert. Die Integration eines Keramikheizers ist nicht erforderlich. Die federnden Kontakte, die ebenfalls durch das MID-Verfahren hergestellt werden, ermöglichen ein Anschließen der Temperiereinrichtung an eine Leiterplatte zur Stromversorgung. Der Einsatz von Pins ist daher nicht notwendig. Der gesamte Herstellprozess der Temperiereinrichtung ist folglich vorteilhafterweise auf die Kunststoffformgebung durch Spritzgießen und das Auftragen der metallischen Struktur auf das MID-Bauteil beschränkt. Die Vorteile dieser Ausführungsform des erfindungsgemäßen Analysegeräts mit Temperiereinrichtung sind eine Vereinfachung des Hezstellprozesses, eine Vermeidung der Probleme durch Keramikbruch, eine Reduzierung der Herstellkosten durch Wegfall der Keramikfertigung, eine dichte Testelementaufnahme und dass komplexe und miniaturisierte Formen möglich sind.

In einem Analysegerät zur Analyse einer Probe auf einem Testelement ist mindestens eine Testelementaufnahme enthalten, um ein Testelement vor und während der Analyse zu positionieren (z. B. bei der Probenaufnahme und insbesondere bei der elektrochemischen oder optischen Analyse der Probe). Gemäß einer Ausführungsform der vorliegenden Erfindung umfasst die Testelementaufnahme mindestens zwei federnde Kontakte, die zur Positionierung eines Testelements in der Testelementaufnahme dienen, wobei die Testelementaufnahme mit den mindestens zwei federnden Kontakten ein spritzgegossener Schaltungsträger (insbesondere ein dreidimensionaler spritzgegossener Schaltungsträger) ist. Die Positionierung kann mit Hilfe einer solchen Testelementaumahme z.B. so erfolgen, dass eine auf dem Testelement vorgesehene metallische, elektrisch leitfähige Struktur (z.B. ein metallisches Feld) die mindestens zwei federnden Kontakte kurzschließt, sobald sich das Testelement in der gewünschten Position befindet. Durch das Kurzschließen kann ein elektrischer Strom über die Kontakte fließen, der detektiert und als Positionssignal ausgewertet werden kann.

Gemäß einer Ausführungsform der vorliegenden Erfindung enthält das erfindungsgemäβe Analysegerät als ein elektrisch kontaktierendes Bauteil, das ein spritzgegossener Schaltungsträger (insbesondere ein dreidimensionaler spritzgegossener Schaltungsträger) ist, ein Kontaktelement zur Testelementauswertung in dem Analysegerät Dieses Kontaktelement zur Testelementauswertung kann ein elektrochemisch auszuwertendes Testelement kontaktieren.

Elektrochemische Verfahren zur Bestimmung der Konzentration eines Analyten beruhen z.B. auf Amperometrie oder Coulometrie. Solche Verfahren sind beispielsweise aus den Druckschriften US 4,654,197, EP 0 505 475 B1 oder US 5,108,564 bekannt. Zur Durchführung der elektrochemischen Analyse müssen zwischen dem Testelement und dem Analysesystem elektrische Signale übertragen werden. Daher muss ein in ein Analysesystem eingebrachtes Testelement in dem Analysesystem mit Hilfe eines elektrischen Anschlusssystems elektrisch kontaktiert werden.

Im Stand der Technik wird für die Kontaktierung ein Steckverbinder als Kontaktelement verwendet, der aus einem Kunststoffteil und aus metallischen Elementen besteht. Das Kunststoffteil dient als Gehäuse und übernimmt die Führungsfunktion für das Testelement Die metallischen Elemente dienen zur Stromleitung und Kontaktierung. Die metallischen Elemente werden durch Biege- und Stanzprozesse hergestellt und werden entweder an das Kunststoffteil montiert oder direkt in dieses eingespritzt. Die Limitationen bei der Konstruktion und Auslegung eines Steckverbinders entstehen hauptsächlich durch die Einschränkungen der Stanz- und Biegeprozesse und durch die Anforderungen der Montierbarkeit bzw. der Umspritzbarkeit der Metallteile. Aufgrund der Stanz- und Biegeprozesse ist die Auslegung und Konstruktion der Kontaktelemente im Stand der Technik im Vergleich zu den Möglichkeiten von Kunststoffprozessen stark eingeschränkt. Zusätzlich müssen bei der Konstruktion die Anforderungen der Montierbarkeit bzw. der Umspritabarkeit der Metallteile berücksichtigt werden. Bei dem erfindungsgemäßen Analysegerät wird die Funktion der elektrischen Kontaktierung nicht durch montierte Metallteile realisiert, sondern es wird ein Kontaktelement durch den Einsatz von MID-Technologie hergestellt

Gemäß einer Ausführungsform der Erfindung enthält das erfindungsgemäße Analysegerät ein Kontaktelement zur Testelementauswertung, das eine Auflagefläche für ein Testelement aufweist, wobei über die Auflagefläche eine Vielzahl von Kontaktrampen hinausragen, die dazu vorgesehen sind, um elektrische Kontakte zu einem auf der Auflagefläche positionierten Testelement herzustellen und die über auf dem Kontaktelement verlaufende Leiterbahnen mit einer Leiterplatte verbindbar sind, wobei das Kontaktelement ein spritzgegossener Schaltungsträger ist, insbesondere ein dreidimensionaler spritzgegossener Schaltungsträger. Vorzugsweise wird ein elektrochemisch zu analysierendes Testelement in einer schlitzfözmigen Führung des Analysegeräts auf der Auflagefläche des Kontaktelements verschoben, bis es auf den Kontaktrampen so positioniert ist, dass diese gegen das Testelement drücken und die elektrischen Kontakte des Testelements kontaktieren. Die Kontaktrampen können z. B. als auf einer Verlängerung der Auflagefläche angeordnete, rampenförmige Erhebungen oder als einzelne rampenförmige, federnde Kontaktfahnen ausgebildet sein. Die Leiterbahnen verlaufen auf dem Kontaktelement z. B. bis zu einem Ende des Kontaktelements, an dem sie mit der Leiterplatte zur Stromversorgung und Signalverarbeitung verlötet werden.

Gemäß einer Ausführungsform der vorliegenden Erfindung ist das Kontaktelement zur Testelementauswertung in das Gehäuse des Analysegeräts integriert, wobei das Gehäuse inklusive Kontaktelement ein spritzgegossener Schaltungsträger ist, insbesondere ein dreidimensionaler spritzgegossener Schaltungsträger.

Gemäß einer anderen Ausführungsform der vorliegenden Erfindung ist das Kontaktelement zur Testelementauswertung in ein Testelementmagazin integriert. Dabei kann entweder für jedes Testelement ein einzelner Kontakt oder eine Kontaktierung für alle im Magazin enthaltenen Testelemente realisiert werden. Am Magazin befindet sich zusätzlich eine Schnittstelle zum Analysegerät. Über diese Schnittstelle werden die Testelemente letztendlich mit dem Gerät verbunden. Diese Ausfübrungsform hat die Vorteile, dass eine bauraumsparende Konstruktion möglich ist, dass keine genaue Positionierung der einzelnen Testelemente zum Analysegerät notwendig ist und dass die Kontaktierung im Magazin kostensparend ausgelegt werden kann, da nur eine einmalige Kontaktierung der beschränkten Anzahl an in dem Magazin enthaltenen Testelementen notwendig ist und das Kontaktelement anschließend mit dem. Magazin entsorgt wird.

Weiterhin wird ein Vorratsbehältnis für mindestens zwei Testelemente (Testelementmagazin) vorgeschlagen, wobei die Testelement elektrische Leiterbahnen aufweisen und das Vorratsbehältnis elektrische Kontakte zur Kontaktierung der Leiterbahnen eines darin enthaltenen Testelements bei der elektrochemischen Analyse einer Probe auf dem Testelement enthält.

Das Vorratsbehältnis ist z. B. ein trommelförmiges Testelementmagazin, das insbesondere weitgehend wie das in DE 198 54 316 A1 beschriebene Vorratsbehältnis aufgebaut sein kann. Das Vorratsbehältnis weist elektrische Kontakte auf, die zur elektrischen Kontaktierung eines elektrochemisch auszuwertenden Testelements dienen. Das Vorratsbehältnis weist vorzugsweise mindestens zwei separate Kammern zum Aufnehmen der Testelemente auf, wobei in jeder Kammer elektrische Kontakte zur Kontaktierung der Leiterbahnen jeweils des darin enthaltenen Testelements bei der elektrochemischen Analyse einer Probe auf dem Testelement angeordnet sind.

Im Stand der Technik erfolgt zumeist die elektrochemische Ausweitung in einem Messmodul des Analysegeräts, in dem das Testelement elektrisch kontaktiert wird und das beabstandet zu dem. Testelementvorratsbehältnis in dem Analysegerät angeordnet ist. Die Testelemente werden bei Bedarf automatisch durch eine Transporteinheit in dem Analysegerät aus dem Testelementvorratsbehältnis befördern, an das Messmodul übergeben und dort zur elektrischen Kontaktierung genau positioniert. Ein Nachteil einer solchen Kontaktierung in einem Messmodul ist z. B., dass ein größere Bauraum innerhalb des Analysegeräts zur elektrochemischen Analyse des Testelement benötigt wird. Ferner muss das Testelement zur Kontaktierung genau positioniert werden. Die Übergabe des Testelements an die Kontaktierungsstelle im Analysegerät ist eine potenzielle Schwachstelle bezüglich Positionierung, Zuverlässigkeit und dynamischer Kontaktierung. Die Kontakte des Messmoduls im Analysengerät sind ferner nur für eine bestimmte Elektrodenstruktur auf Testelementen geeignet.

Die vorgeschlagene Lösung hat hingegen den sich aus der Integration der elektrischen Kontaktierung in das Vorratsbehältnis (Testelementmagazin) ergebenden. Vorteil, dass eine einfache standardisierbare Schnittstelle wischen Analysegerät und Magazin möglich ist, wobei die z. B. in den Kammern des jeweiligen Magazins angeordneten elektrischen Kontakte auf die Elektrodenstruktur der darin enthaltenen Testelement abgestimmt und variabel gestaltbar sind. Es ist ferner eine bauraumsparende Konstruktion von. Analysegerät und Magazin möglich. Des Weiteren ist keine genaue Positionierung des Testelements außerhalb des Vorratsbehältnisses im Analysegerät erforderlich. Die Kontaktierung der Testelemente im Magazin kann kostensparend ausgelegt werden, da diese nur die in dem Magazin enthaltenen Testelemente kontaktieren muss und das Magazin anschließend entsorgt werden kann.

Die Kontaktierung im Vorratsbehältnis kann über montierte oder umspritzte Metallteile realisiert werden. Gemäß einer bevorzugten Ausführungsform ist das Vorratsbehältnis mit den elektrischen Kontakten im Wesentlichen ein spritzgegossener Schaltungsträger, insbesondere ein dreidimensionaler spritzgegossener Schaltungsträger.

Im Stand der Technik wird zumeist bei elektrochemisch auszuwertenden Testelementen die notwendige Elektrodenstruktur meist über ein Druckverfahren oder durch Laserablation hergestellt. Dazu wird zunächst das Testelement produziert und anschließend die Elektrodenstruktur aufgebracht. Eine dreidimensionale Gestaltung des Testelements ist dadurch stark eingeschränkt bzw. nicht möglich. Daher sind größere Testelemente mit Mehrfachtests oder Tests für verschiedene Parameter schwer herstellbar, da bei der Laserablation die Größe des Belichtungsfensters eingeschränkt ist und beim Druckverfahren die Gestaltungsmöglichkeiten sehr stark limitiert sind.

Vorgeschlagen wird daher auch ein Testelement, enthaltend ein Testfeld zur elektrochemischen Analyse einer flüssigen Probe in einem Analysengerät, wobei das Testfeld auf dem Testelement mit elektrischen Leiterbahnen verbunden ist, wobei das Testelement mit den elektrischen. Leiterbahnen im Wesentlichen ein spritzgegossener Schaltungsträger ist.

Bei der vorgeschlagenen Lösung wird die Elektrodenstruktur mittels eines MID-Verfahrens in einen Kunststoffprobenträger integriert. Dadurch ergeben sich die Vorteile einer großen konstruktiven Freiheit bei der Gestaltung des Testelements (echte 3D-Strukturen möglich), eines einfachen Herstellungsprozesses (Spritzguss) und, dass die Herstellung großer Testelemente mit vielen Testbereichen möglich ist.

Die Erfindung bezieht sich weiterhin auf ein Verfahren zur Herstellung eines Analysegeräts zur Analyse einer Probe auf einem Testelement, enthaltend mindestens ein elektrisch kontaktiErendes Bauteil zur Stromübertragung, das zur Herstellung eines elektrischen Kontakts zu mindestens einem weiteren kontaktierten Bauteil geeignet ist, wobei das elektrisch kontaktierende Bauteil mindestens eine federnde Kontaktfahne zur Kontaktierung des weiteren Bauteils aufweist, die einen Kontaktfahnenkörper aus spritzgegossenem Kunststoff und eine metallische Stromleiterstruktur enthält, wobei die Kontaktfahne ein rotierendes oder ein linear bewegtes weiteres Bauteil des Analysegeräts kontaktiert, wobei das Verfahren folgende Schritte aufweist:
a) Herstellen des elektrisch kontaktierenden Bauteils, das einen Grundkörper und eine metallische Leiterstruktur umfasst, durch ein Verfahren zur Herstellung spritzgegossener Schaltungsträger (MID), insbesondere dreidimensionaler spritzgegossener Schaltungsträger, und
b) Positionieren und Montieren des elektrisch kontaktierenden Bauteils in dem Analysegerät zur Herstellung eines elektrischen Kontakts zu dem weiteren kontaktierten Bauteils.

Das Verfahren zur Herstellung spritzgegossener Schaltungsträger (MID), insbesondere dreidimensionaler spritzgegossener Schaltungsträger, ist dabei vorzugsweise ein Verfahren, ausgewählt aus der Gruppe Zweikomponentenspritzgießen, Heißprägen, Folienhinterspritzen und Laserstrukturierung.

Die Erfindung wird nachstehend anhand der Zeichnung näher erläutert.

Es zeigt:
- Figur 1.1: ein Analysegerät aus dem Stand der Technik,
- Figur 1.2: eine Explosionsdarstellung des Analysegeräts aus Figur 1.1,
- Figur 2: eine Explosionsdarstellung verschiedener Baugruppen eines Analysegeräts aus dem Stand der Technik,
- Figur 3.1: eine Explosionsdarstellung einer Positioniereinrichtung für ein Testelementmagazin in einem Analysengerät aus dem Stand der Technik,
- Figur 3.2: eine Positioniereinrichtung für ein Testelementmagazin in einem erfindungsgemäßen Analysegerät,
- Figur 4.1: eine Transporteinheit für Testelemente in einem Analysengerät aus dem Stand der Technik,
- Figur 4.2: eine Transporteinheit für Testelemente in einem erfindungsgemäßen Analysegerät,
- Figur 5.1: eine Explosionsdarstellung eines Motormoduls in einem Analysegerät aus dem Stand der Technik,
- Figur 5.2: einen Motorhalter eines Motormoduls in einem Analysegerät,
- Figur 6.1: eine Barcodereaderanordnung in einem. Analysegerät aus dem Stand der Technik,
- Figur 6.2: eine Barcodereaderanordnung in einem Analysegerät,
- Figur 7.1: eine Testelementmagazinaufnahme in einem Analysegerät aus dem Stand der Technik,
- Figur 7.2: eine Testelementmagazinaufnahme mit einem Sensor in einem Analysegerät,
- Figur 8: ein Kontaktelement zur Testelementauswertung in einem Analysegerät aus dem Stand der Technik,
- Figur 9: ein erstes Kontaktelement zur Testelementauswertung in einem Analysegerät,
- Figur 10: ein zweites Kontaktelement zur Testelementauswertung in einem An al ysegerät,
- Figur 11.1: eine Temperiereinrichtung in einem Messmodul eines Analysegeräts aus dem Stand der Technik,
- Figur 11.2: die Rückseite eines Heizelements aus dem Stand der Technik,
- Figur 11.3: die Vorderseite eines Heizelements aus dem Stand der Technik,
- Figur11.4: eine Temperiereinrichtung in einem Messmodul eines Analysegeräts,
- Figur 12: ein Vorratsbehältnis für Testelemente in einem Analysengerät und
- Figur 13: eine erste Ausführungsform eines im Wesentlichen als spritzgegossener Schaltungsträger ausgeführten Testelements und
- Figur 14: eine zweite Ausführungsform eines im Wesentlichen als spritzgegossener Schaltungsträger ausgeführten Testelements.

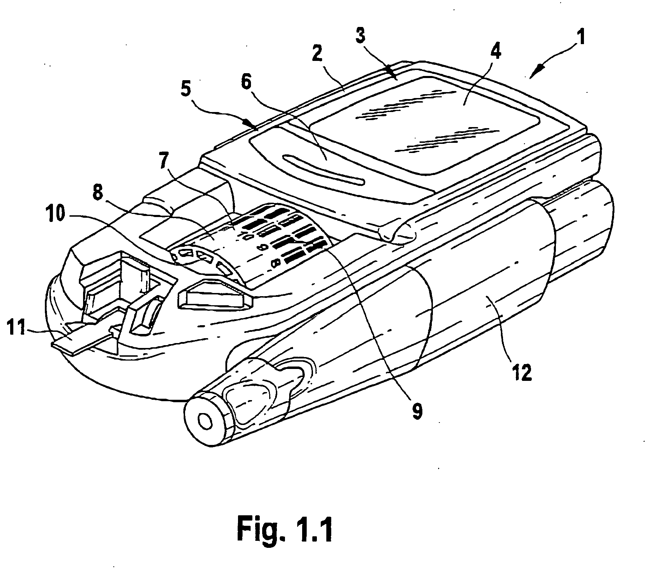

Figur 1.1 zeigt ein Analysegerät aus dem Stand der Technik,

Das Analysegerät 1 weist ein Gehäuse 2 auf, das einen Anzeigebereich 3 (LCD 4) und einen Bedienbereich 5 (Einschalttaster 6) enthält. Bei dem in Figur 1.1 dargestellten Analysengerät 1 ist die Testelementmagazinaufnahme 7 sichtbar, da eine Testelementmagazinaufnahmeabdeckung entfernt wurde. In der Testelementmagazinaufnahme 7 befindet sich ein trommelförmiges Testelementmagazin 8, das eine Vielzahl von Testelementen aufnehmen kann. An der Außenseite weist das Testelementmagazin einen Barcode 9 auf, der Informationen, z. B. über die darin enthaltenen Testelemente, enthält und der von einem (nicht dargestellten) Barcodereader eingelesen werden kann. Über den Einschalttaster 6 wird ein Messvorgang gestartet. Ein (nicht sichtbarer) Motor dreht das Testelementmagazin 8 um eine Kammer 10 weiter und ein zweiter (nicht sichtbarer) Motor schiebt mit Hilfe einer Schubstange ein Testelement 11 aus einer Kammer 10 des Testelementmagazins 8 aus, bis es aus dem Analysegerät 1 herausragt. In dieser Position kann ein Anwender eine Probe (z. B. Blut) auf das Testelement 11 geben. Eine (nicht sichtbare) Messoptik in dem Analysegerät 1 analysiert die Probe auf dem Testelement 11. Auf dem LC-Display wird ein Ergebnis der Analyse (z. B. ein Blutzuckerwert) angezeigt. Seitlich am Gehäuse 2 des Analysegerät 1 ist eine abnehmbare Stechhilfe 12 angebracht, die zur Probengewinnung genutzt werden kann.

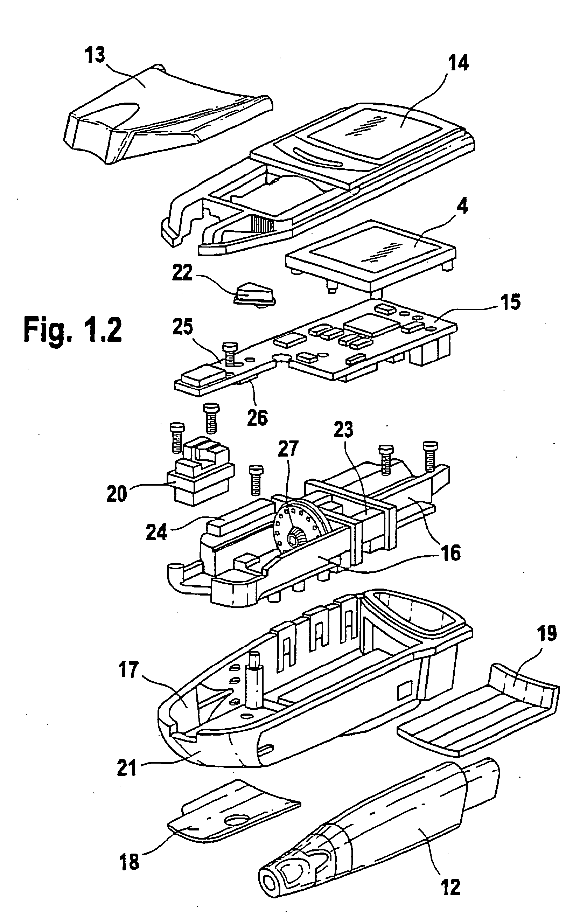

Figur 1.2 zeigt eine Explosionsdarstellung des Analysegeräts aus Figur 1.2.

Von oben nach unten sind die Testelementmagazinaufnahmeabdeckung 7, die obere Gehäusehälfte 14, das LC-Display 4, die Leiterplatte 15, das Messmodul 20, das Motormodul 16, die untere Gehäusehälfte 17, das Typenschild 18, der Batteriefachdeckel 19 und die Stechhilfe 12 dargestellt. Die untere Gehäusehälfte 17 weist einen aufklappbaren Deckel 21 auf, der sich mit Hilfe des Entriegelungsknopfes 22 entriegeln und öffnen lässt. Dann kann ein Testelementmagazin in die Testelementmagazinaufnahme 7 eingesetzt oder ausgetauscht werden. Das Motormodul 16 umfasst ein Schubstangenmotormodul 23 und ein Magazinmotormodul 24. Das Magazinmotormodul 24 weist unter anderem das in die Testelementmagazinaufnahme 7 hineinreichende Antriebsrad 27 auf, das für einen Eingriff in ein anzutreibendes Testelementmagazin eine Verzahnung hat. Die Leiterplatte 15 hat einen bis in die Testelementmagazinaufnahme 7 hineinreichenden Leiterplattenarm 25, an dem unter anderem ein Barcodereader 26 befestigt ist.

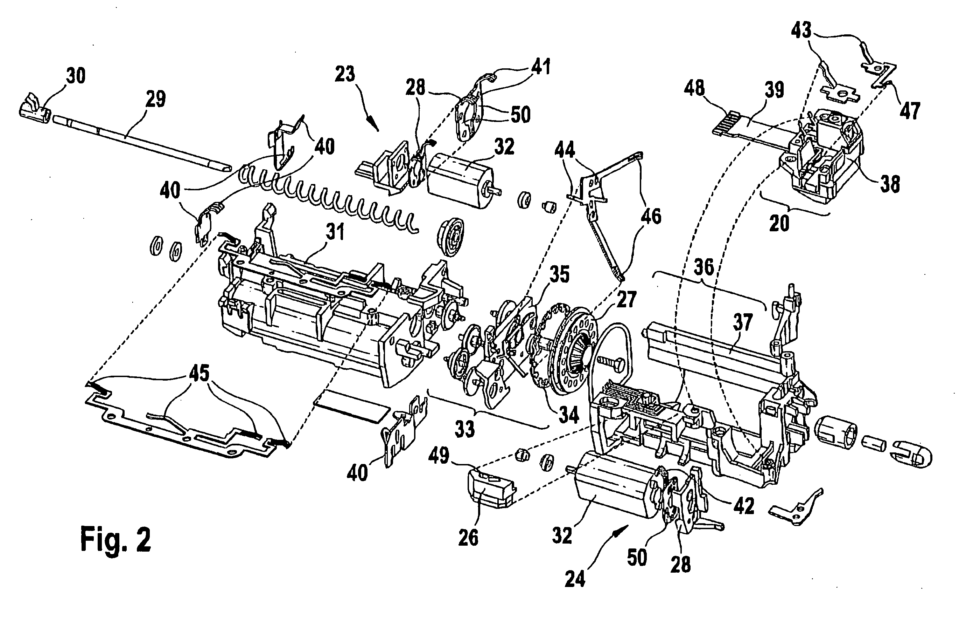

Figur 2 zeigt eine Explosionsdarstellung verschiedener Baugruppen eines Analysegeräts aus dem Stand der Technik.

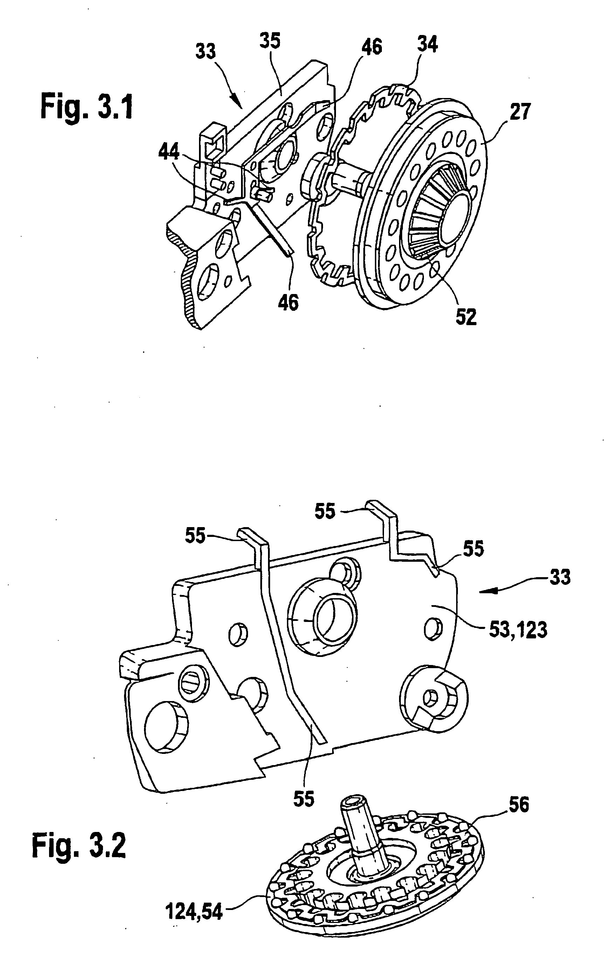

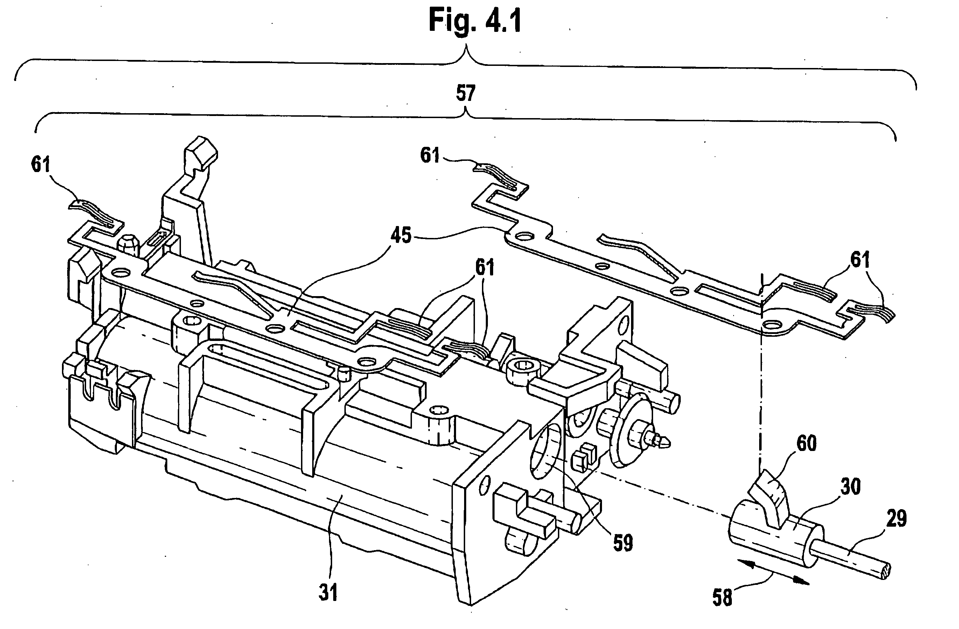

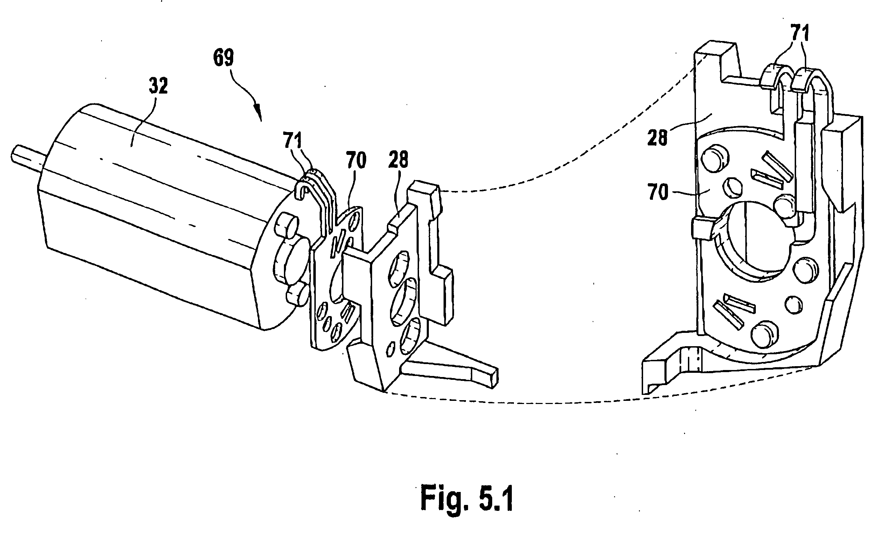

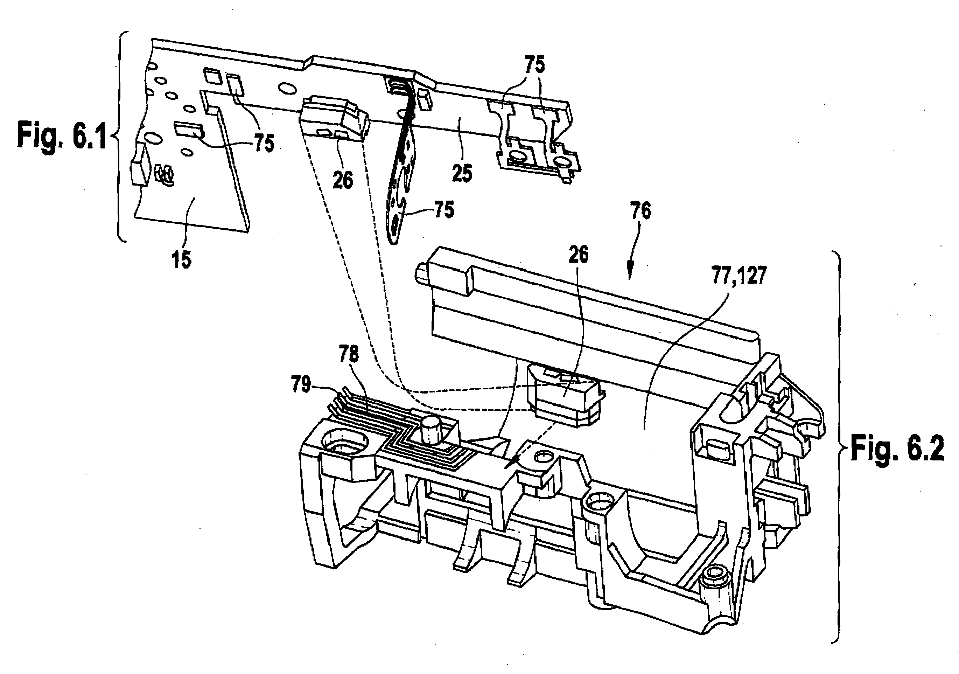

Die verschiedenen Baugruppen weisen eine Vielzahl von elektrischen Kontakten zur Stromübertragung auf. Zur Klassifizierung werden federnde Kontakte, Steckkontakte, Lötkontakte und Schleifkontakte unterschieden. Zur elektronischen Differenzierung werden diese Kontakte nach der Strombelastung in Signal- und Leistungsstromübertragung eingeteilt. Weiterhin werden die federnden Kontakte mechanisch in statisch einfedernde und dynamisch einfedernde Kontakte unterteilt. Figur 2 zeigt die verschiedenen Kontaktarten, die die Baugruppen Motormodul 16, Messmodul 20 und Barcodereader 26 miteinander und mit der (nicht dargestellten) Leiterplatte verbinden. Im Einzelnen sind in Figur 2 folgende Bauteile dargestellt:
a) Eine Transporteinheit für Testelemente umfasst eine Schubstange 29, eine Führungsbuchse 30 und eine Führungseinrichtung 31, in der die Führungsbuchse 30 führbar ist. Die Funktionsweise der Transporteinheit wird anhand von Figur 4.1 näher erläutert.
b) Ein Schubstangenmotormodul 23 umfasst einen Motorhalter 28 und einen Motor 32.
c) Ein Magazinmotormodul 24 umfasst ebenfalls einen Motorhalter 28 und einen Motor 32. Die Motormodule werden anhand von Figur 5.1 naher erläutert.
d) Eine Positioniereinrichtung 33 für ein Testelementmagazin umfasst ein Antriebsrad 27, eine Segmentscheibe 34 und eine Getriebeplatine 35. Die Positioniereinrichtung wird anhand von Figuren 3.1 und 3.2 näher erläutert.
e) Eine Barcodereaderanordnung 36 umfasst einen Gehäuseabschnitt 37 und einen Barcodereader 26, der an einem (nicht dargestellten) in den Gehäuseabschnitt 37 hineinragenden Leiterplattenann befestigt ist Die Barcodereaderanordnung 36 wird anhand von Figur 6.1 näher erläutert.
f) Ein Messmodul 20 umfasst eine Testelementaufnahme 38 und eine Optikplatine 39.
   Die Baugruppen weisen unter anderem die folgenden elektrischen Kontakte auf: Batteriekontakte 40, Magazinmotorkontakt 41, Schubstangenmotorkontakt 42, Messmodulkontakt 43, Positionsschalterkontakte 44 des Antriebsrads 27, Schubstangenschalterkontakte 45, Schleifkontakte 46 zum Antriebsrad 27, Testelementkontakt 47 im Messmodul 20, Optikplatinenkontakt 48 im Messmodul 20, Barcodereaderkontakt 49, Motorkontakte 50 und Magazinpositionskontakt 51 in Form der Segmentscheibe 34.

In diesem Analysegerät aus dem Stand der Technik sind mehr als 67 Kontakte vorhanden, die durch mehr als 19 separate Bauteile realisiert werden. In dem erfindungsgemäßen Analysegerät können diese separaten Bauteile und die damit verbundenen Montageschritte weitgehend eingespart werden, indem sie in elektrisch kontaktierende MID-Bauteile integriert werden.

Figur 3,1 zeigt eine Explosionsdarstellung einer Positioniereinrichtung für ein Testelementmagazin in einem Analysegerät aus dem Stand der Technik.

Die Positioniereinrichtung 33 umfasst eine Getriebeplatine 35, eine metallische Segmentscheibe 34 und ein Antriebsrad 27. Das Antriebsrad 27 wird mit Hilfe eines (nicht dargestellten) Motors gedreht. Durch die Verzahnung 52 am Antriebsrad 27 wird ein (nicht dargestelltes) Testelementmagazin mitgedreht. Die Drehung des Testelementmagazins wird im Stand der Technik durch die zwei auf der Getriebeplatine 35 heißverstemmten Schleifkontakte 46 auf der Segmentscheibe 34 erfasst. Durch zwei weitere Positionsschalterkontakte 44 wird das Signal zu einer (nicht dargestellten) Leiterplatte geleitet.

Figur 3.2 zeigt eine Positioniereinrichtung 33 für ein Testelementmagazin in einem erfindungsgemäßen Analysegerät. Diese Positioniereinrichtung umfasst statt fünf separaten nur zwei Bauteile: Die Platine 53 (elektrisch kontaktierendes Bauteil 123), die ein spritzgegossener Schaltungsträger ist, und das Antriebsrad 54 (elektrisch kontaktierendes Bauteil 124), das ebenfalls ein spritzgegossener Schaltungsträger ist. In die MID-Platine 53, 123 sind Federkontakte 55 integriert, die zum Kontaktieren der Segmentscheibe 56 und einer weiteren (nicht dargestellten) Leiterplatte dienen. Die Segmentscheibe 56 ist in das Antriebsrad 54, 124 integriert.

Figur 4.1 zeigt eine Transporteinheit für Testelemente in einem Analysegerät aus dem Stand der Technik.

Zu der Transporteinheit 57 gehört eine Schubstange 29, mit der ein Testelement in der Axialrichtung 58 bewegt werden kann. Die Schubstange 29 wird durch einen (nicht dargestellten) Schubstangenmoter angetrieben. Die Schubstange 29 weist eine Führungsbuchse 30 auf, die in einer oben offenen Längsbohrung 59 der Führungseinrichtung 31 geführt wird. An der Führungsbuchse 30 ist ein nach oben ragender Abstandshalter 60 angebracht. Auf der Führungseinrichtung 31 ist ein als metallenes Stanz- und Biegeteil ausgeführter Schubstangenschalterkontakt 45 befestigt. Die drei Kontaktfahnen 61 des Schubstangenschalterkontakts 45 werden bei drei verschiedenen Positionen der Fübrungsbuchse 30 in der Längsbohrung 59 durch den Abstandshalter 60 nach oben gegen die Kontaktflächen einer (nicht dargestellten) Leiterplatte gedrückt, so dass die jeweilige Position erkannt wird.

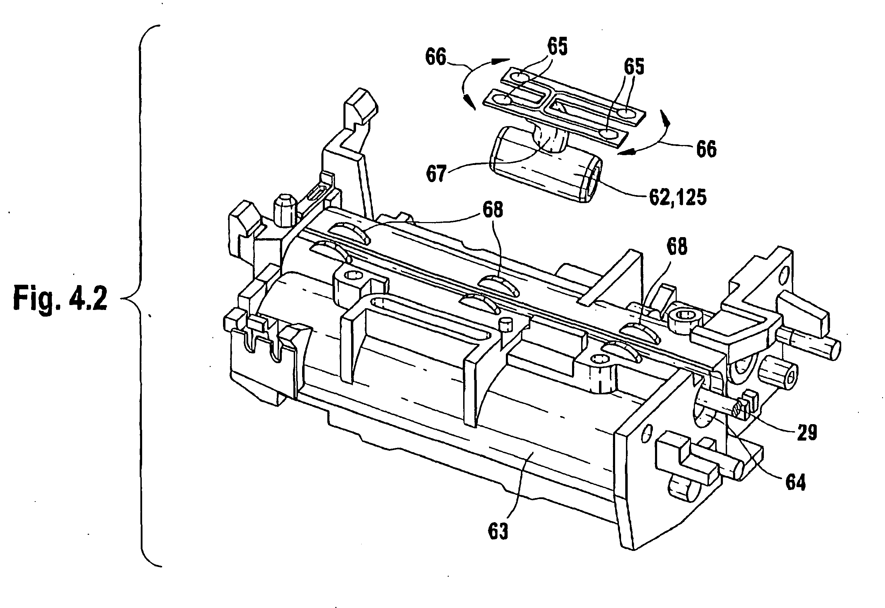

Figur 4.2 zeigt eine Transporteinheit für Testelemente in einem erfindungsgemäßen Analysegerät.

Auch diese Transporteinheit umfasst eine Führungsbuchse 62 für die (nicht dargestellte) Schubstange 29 und eine darauf abgestimmte Führungseinrichtung 63. Die Führungseinrichtung 63 enthält eine nach oben offene Längsbohrung 64, in der die Führungsbuchse 62 geführt wird. Die Führungsbuchse 62 weist Kontaktfahnen 65 auf, die in der Richtung 66 federnd ausgebildet sind. Die Führungsbuchse 62 (elektrisch kontaktierendes Bauteil 125) mit den über einen Abstandhalter 67 verbundenen Kontaktfahnen 65 ist ein spritzgegossener Schaltungsträger (MID). Die Führungseinrichtung 63 ist ein Spritzgussteil und weist Schaltelemente 68 auf, die in drei verschiedenen Positionen der Führungsbuchse 62 die Kontaktfahnen 65 gegen Kontaktflächen einer (nicht dargestellten) Leiterplatte nach oben drücken. Somit können diese drei Positionen von dem Analysegerät erkannt werden.

Figur 5.1 zeigt eine Explosionsdarstellung eines Motormoduls in einem Analysegerät aus dem Stand der Technik.

Das Motormodul 69 kann z. B. ein Schubstangenmotormodul oder ein Magazinmotormodul sein. Es umfasst einen Motor 32 und einen Motorhalter 28. Zur Stromversorgung des Motors 32 wird in dem Motorhalter 28 ein Kontaktblech 70 eingepresst, das über federnde Kontaktfahnen 71 eine elektrisch leitende Verbindung zu einer (nicht dargestellten) Leiterplatte herstellt.

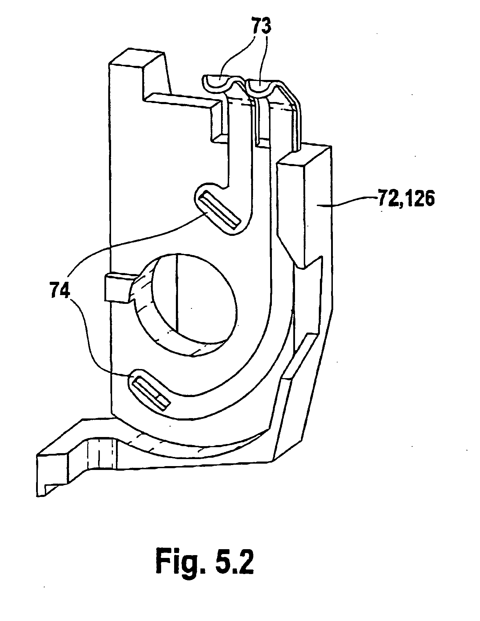

Figur 5.2 zeigt einen Motorhalter eines Motormoduls in einem Analysegerät.

Der Motorhalter 72 (elektrisch kontaktierendes Bauteil 126) ist ein spritzgegossener Schaltungsträger (MID). In dieses elektrisch kontaktierende MID-Bauteil sind der Motorhalter 72 und die elektrischen Kontakte in Form von Kontaktfahnen 73 mit den davon ausgehenden Leiterbahnen 74 integriert. Die Kontaktfahnen 73 können an eine Leiterplatte gelötet werden.

Figur 6.1 zeigt eine Barcodereaderanordnung in einem Analysegerät aus dem Stand der Technik.

Ein Barcodereader 26 ist an einem Leiterplattenarm 25 der Leiterplatte 15 befestigt, wird über die Leiterplatte 15 mit Strom versorgt und führt Signale über diese ab. Der Leiterplattenarm 25 weist ferner weitere Bauteile 75 auf, die nicht näher erläutert werden sollen. Der Barcodereader 26 ist so an dem Leiterplattenarm 25 angeordnet, dass er in eine (nicht dargestellte) Testelementmagazinaufnahme hineinragt, um dort den Barcode auf dem jeweils aufgenommenen Testelementmagazin einlesen zu können.

Figur 6.2 zeigt eine Barcodereaderanordnung in einem Analysegerät.

Die Barcodereaderanordnung 76 umfasst einen Gehäuseabschnitt 77 des Analysegeräts, in dem ein Barcodereader 26 angeordnet ist. Der Gehäuseabschnitt 77 enthält zu dem Barcodereader 26 verlaufende Leiterbahnen 78 und federnde Kontakte 79 zum Kontaktieren einer (nicht dargestellten) Leiterplatte. Der Gehäuseabschnitt 77 (elektrisch kontaktierendes Bauteile 127) mit den Leiterbahnen 78 und den federnden Kontakten 79 ist ein dreidimensionaler spritzgegossener Schaltungsträger. Da die Stromleitung zu und von dem. Barcodereader 26 in dieser Ausführungsform durch den MID-Gehäuseabschnitt 77 erfolgt, kann (vorausgesetzt, für die weiteren Bauteile 75 in Figur 6.1. wird ebenfalls eine andere Lösung gefunden) der Leiterplattenarm. 25 aus dem Stand der Technik (siehe Figur 6.1) entfallen. Dadurch wird Bauraum im Analysegerät gewonnen und die Leiterplatte kann in günstigeren Losen und mit weniger Verschnitt hergestellt werden.

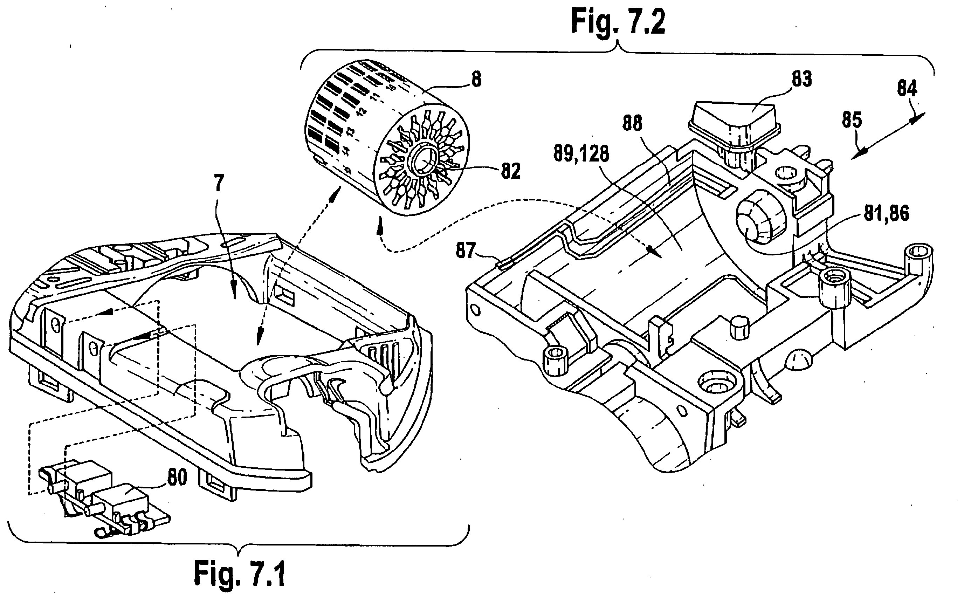

Figur 7.1 zeigt eine Testelementmagazinaufnahme in einem Analysegerät aus dem Stand der Technik.

In die Testelementmagazinaufnahme 7 kann ein Testelementmagazin 8 eingesetzt werden. Ein Wechsel des Testelementmagazins 8 wird in dem Analysegeräte aus dem Stand der Technik durch ein Öffnen und Schließen einer (nicht dargestellten) Testelementmagazinaufnahmeabdeckung registriert, wodurch ein Verriegelungsschalter 80 betätigt wird. Wird die Testelementmagazinaufnahmeabdeckung ohne ein Wechseln des Testelementmagazins 8 geöffnet und geschlossen, so registriert das Analysegerät fälschlicherweise einen Wechsel des Testelementmagazins 8.

Figur 7.2 zeigt eine Testelementmagazinaufnahme mit einem. Sensor zum Erkennen eines Testelementmagazinwechsels in einem Analysegerät.

In dem Analysegerät wird ein Wechsel des Testelementmagazins 8 erkannt, wenn eine Dornhülse 81, die das Testelementmagazin 8 an der Stelle 82 abstützt, durch einen Riegel 83 aus der Arretierung gelöst wurde, sich in Öffnungsrichtung 84 bewegt hat und nach Schließen der Testelementmagazinabdeckung wieder in Schließrichtung 85 bewegt wurde. Ein. Sensor 86 zur Erkennung dieses Bewegungsablaufs ist z. B. so gestaltet, dass ein (nicht dargestellter) Schaltnocken an der Dornhülse 81 einen Kontakt schließt und das Signal über Federkontakte 87 zur (nicht dargestellten) Leiterplatte weiterleitet. Dadurch kann eine fehlerhafte Erkennung eines Wechsels des Testelementmagazins 8 weitgehend vermieden werden. Die Testelementmagazinaufnahme 89 (elektrisch kontaktierendes Bauteil 128) enthält in dieser Ausführungsform die Federkontakte 87 und Leiterbahnen 88, die die Federkontakte 87 mit dem Sensor 86 verbinden, und ist ein dreidimensionaler spritzgegossener Schaltungsträger (MID).

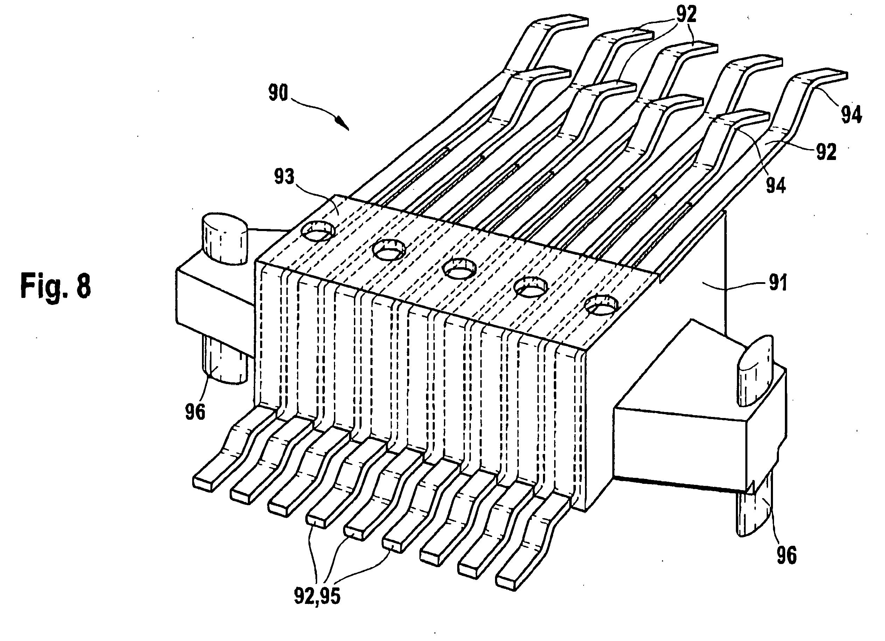

Figur 8 zeigt ein Kontaktelement zur Testelementauswertung in einem Analysegerät aus dem Stand der Technik.

Das Kontaktelement 90 dient zur elektrischen Kontaktierung eines (nicht dargestellten) elektrochemisch auszuwertenden Testelements. Das Kontaktelement 90 ist aus einem Kunststoffteil, 91 und metallischen Elementen 92 aufgebaut. Die metallischen Elemente 92 werden durch Biege- und Stanzprozesse hergestellt, an dem Kunststoffteil 91 montiert oder direkt eingespritzt. Zur Kontaktierung wird ein Testelement auf der Auflagefläche 93 in Richtung der Kontaktrampen 94 verschoben, bis die Kontaktrampen 94 gegen auf dem Testelement vorhandene Kontaktflächen drücken und dieses so kontaktierten. Die Kontaktfahnen 95 sind im Analysegerät an der Leiterplatte festgelötet. Die Stecker 96 denen zur Ausrichtung des Kontaktelements 90 bei seiner Montage im Analysegerät. Bei der Konstruktion und Auslegung dieses Kontaktelements aus dem Stand der Technik bestehen Limitationen durch Einschränkungen der Stanz- und Biegeprozesse und durch Anforderungen der Montierbarkeit und Umspritzbarkeit der metallischen Elemente.

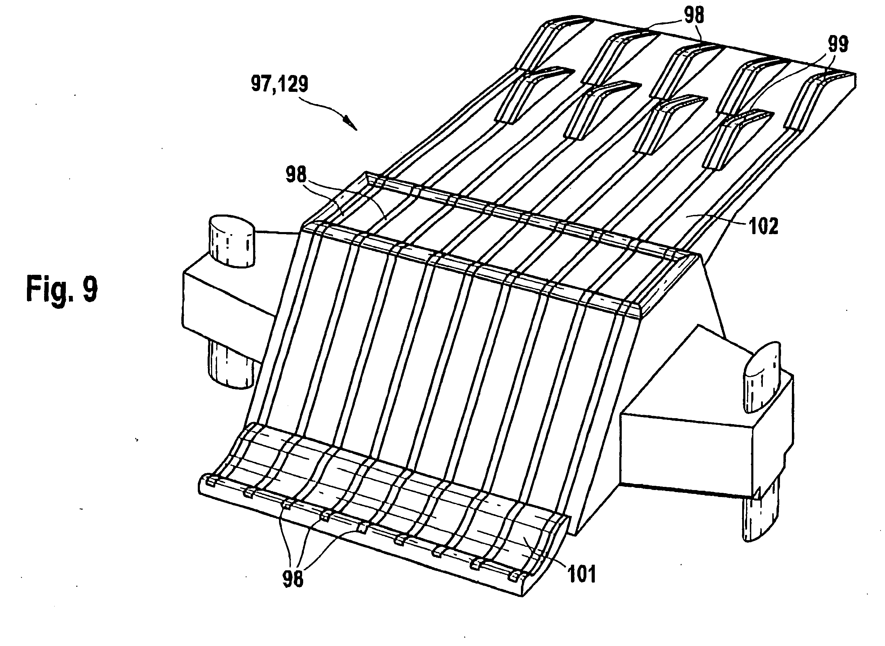

Figur 9 zeigt ein erstes Kontaktelement zur Testelementauswertung in einem Analysegerät.

Dieses Kontaktelement 97 (elektrisch kontaktierendes Bauteil 129) ist ein dreidimensionaler spritzgegossener Schaltungsträger (MID). Die Leiterbahnen 98 und Kontaktrampen 99 sind als elektrisch leitfähige Bereiche direkt auf dem spritzgegossenen Kunststoffteil 100 verwirklicht. Die Leiterbahnen 98 verlaufen von einer mit einer Leiterplatte verbindbaren Kontaktfahne 101 über eine Testelementauflagefläche 102 bis auf die Kontaktrampen 99. Die Kontaktrampen 99 werden durch Kunststofferhebungen auf einer Fläche des Kunststoffteils 100 gebildet, wobei auf den Kunststofferhebungen die metallischen Leiterbahnen 98 verlaufen.

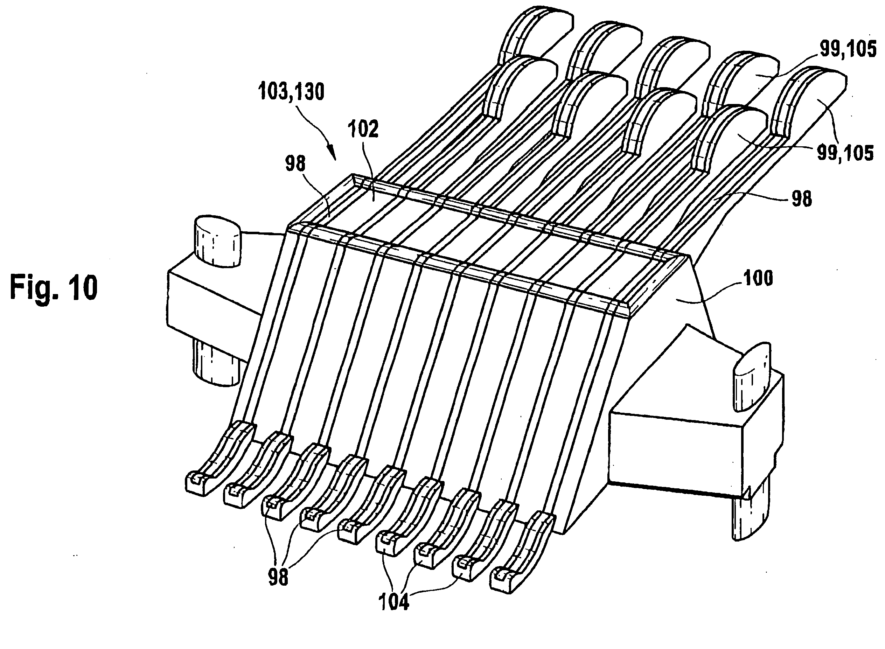

Figur 10 zeigt ein zweites Kontaktelement zur Testelementauswertung in einem Analysegerät.

Dieses Kontaktelement 103 (elektrisch kontaktierendes Bauteil 130) ist ebenfalls ein dreidimensionaler spritzgegossener Schaltungsträger (MID) mit Leiterbahnen 98 und Kontaktrampen 99 auf einem spritzgegossenen Kunststoffteil 100, das eine Testelementauflagefläche 102 aufweist. Im Unterschied zu der in Figur 9 gezeigten. Ausführungsform sind dabei separate Kontaktfahnen 104 und separate Kontaktrampen 105 für jede der Leiterbahnen 98 vorgesehen.

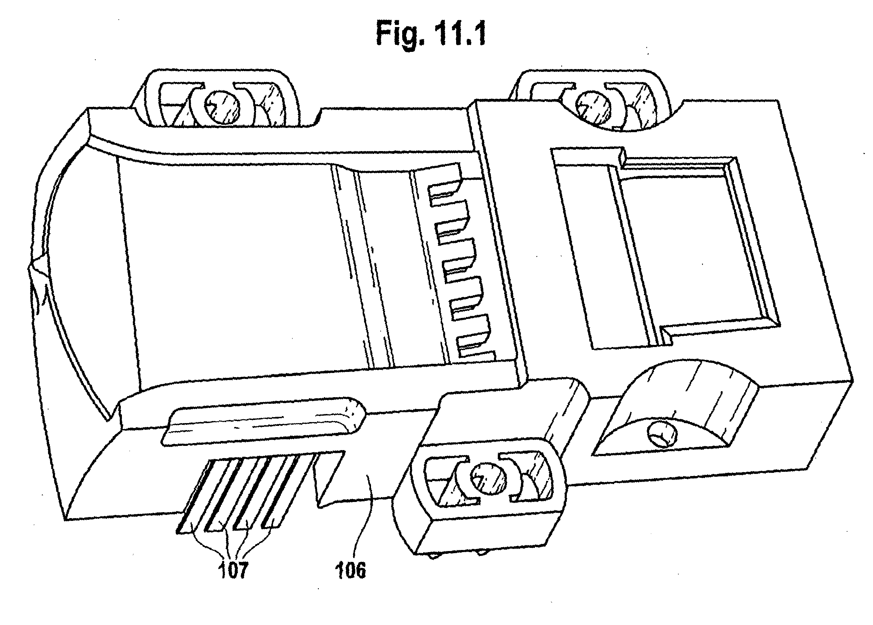

Figur 11.1 zeigt eine Temperiereinrichtung in einem Messmodul eines Analysegeräts aus dem Stand der Technik.

In einer Analysegerätgehäusehälfte 106 ist ein Keramikheizelement montiert oder direkt beim kierstellungsprozess eingearbeitet. Die elektrischen Anschlüsse 107 des Keramikheizelements sind in Figur 11.1 sichtbar.

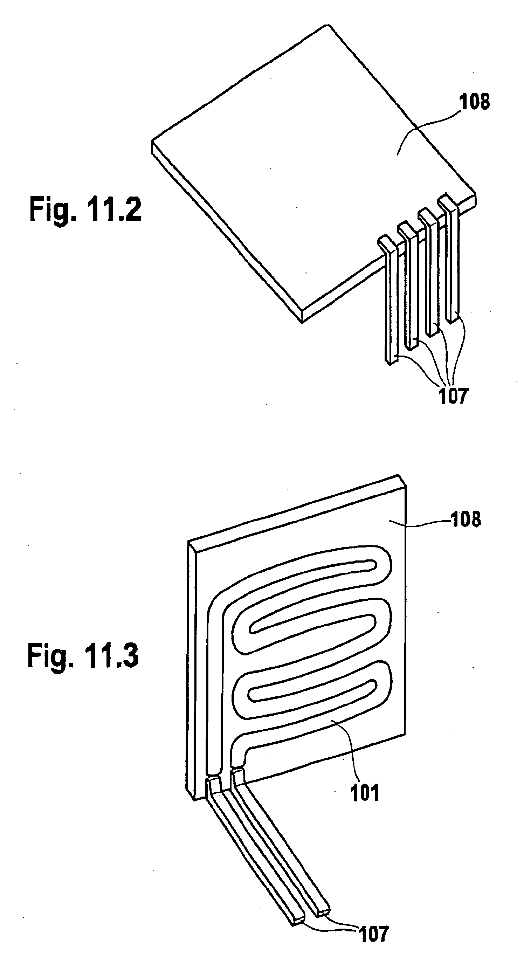

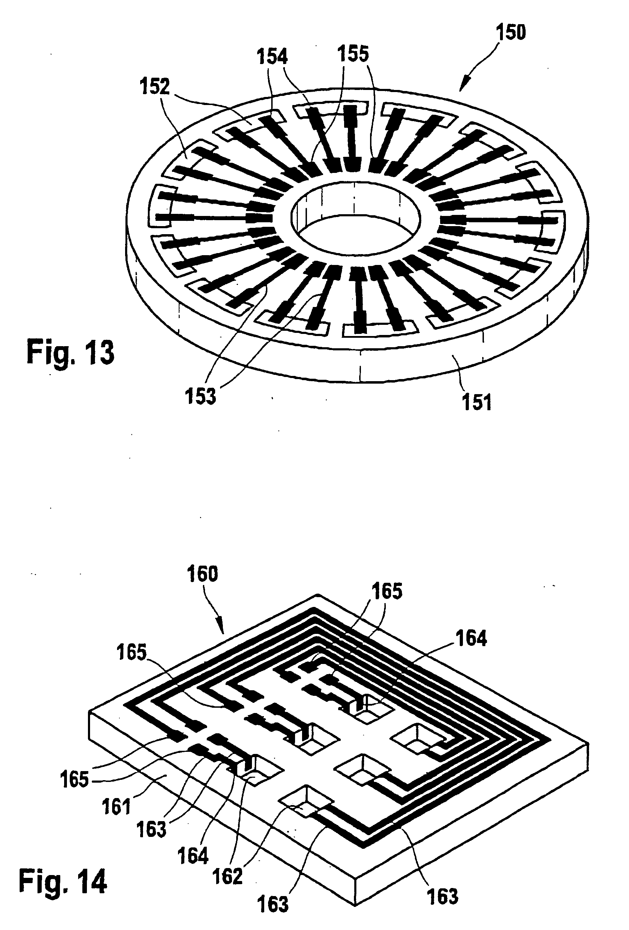

Das keramische Heizelement 108 ist von der Rückseite in Figur 11.2 und von der Vorderseite in Figur 13 zu sehen. Der Einfachheit halber sind in Figur 11.3 nur zwei der vier elektrischen Anschlüsse 107 dargestellt. Die zwei Anschlüsse 107 sind mit einer Heizwendel 109 auf einer keramischen Platte 110 verbunden.

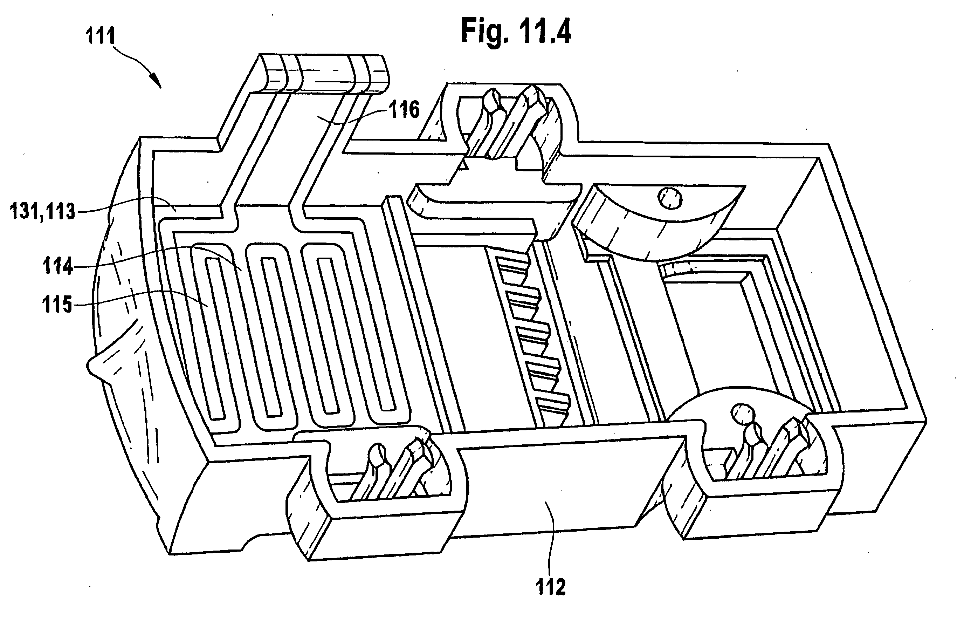

Figur 11.4 zeigt eine Temperiereinrichtung in einem Messmodul eines Analysegeräts.

Das Messmodul 111 ist in der Analysegerätgehäusehälfte 112 angeordnet und umfasst eine Testelementaufnahme 113, in die eine Temperiereinrichtung 114 integriert ist. Die Testelementaufnahme 113 (elektrisch kontaktierendes Bauteil 131) enthält Heizwendeln 115 und einen federnden Kontakt 116 und ist ein dreidimensionaler spritzgegossener Schaltungsträger.

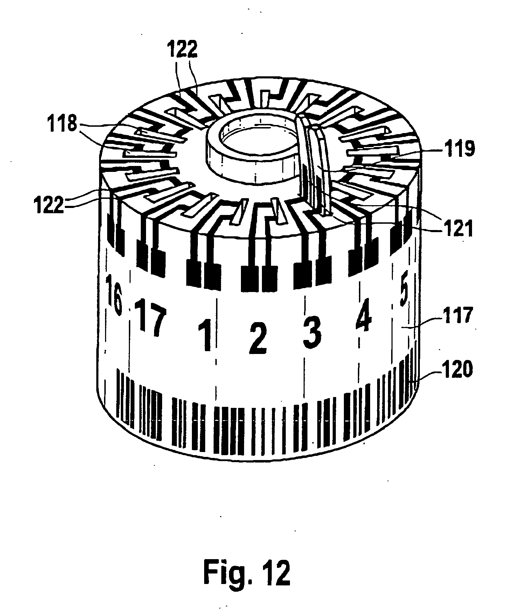

Figur 12 zeigt ein Vorratsbehältnis für Testelemente in einem Analysegerät.

Das Vorratsbehältnis 117 ist ein trommelförmiges Testelementmagazin mit 17 separaten Kammern. 118 zur Aufnahme von 17 streifenförmigen. Testelementen 119. Das Vorratsbehältnis 117 weist an seiner Außenseite einen Barcode 120 auf. Die Testelement 119 sind elektrochemisch auszuwertende Testelemente 119, die mit elektrischen Leiterbahnen 121 versehen sind. Das Vorratsbehältnis 117 enthält in jeder Kammer 118 elektrische Kontakte 122 zur Kontaktierung der Leiterbahnen 121. der darin enthaltenen Testelemente 119 bei der elektrochemischen. Analyse einer Probe auf dem Testelement. Das in Figur 12 dargestellte, aus dem Vorratsbehältnis 117 zum Teil herausragende Testelement 119 ist leicht gebogen, so dass die darauf angeordneten Leiterbahnen 121 gegen die elektrischen Kontakte 122 des Vorratsbehältnisses 117 gedrückt werden und so eine Kontaktierung erreicht wird. Die Kontaktierung kann jedoch auch erfolgen, wenn das Testelement 119 vollständig in dem Vorratsbehältnis 117 steckt.

Zur Kontaktierung sind z. B. auch federnde Kontakten in den Kammern 118 möglich. Das Vorratsbehältnis 117 (elektrisch kontaktierendes Bauteil 132) kann z. B. ein dreidimensionaler spritzgegossener Schaltungsträger (MID) sein.

Figur 13 zeigt eine erste Ausführungsform eines im Wesentlichen als spritzgegossener Schaltungsträger ausgeführten Testelements.

Das Testelement 150 weist einen. Grundkörper 151 aus Kunststoff auf. Auf dem Grundkörper 151 ist eine Vielzahl von Testfeldern 152 zur elektrochemischen Analyse einer flüssigen Probe angeordnet. Auf den. Testfeldern 152 befindet sich eine Trockenchemie, die mit der flüssigen Probe reagiert. Die Testfelder 152 sind auf dem Testelement 150 jeweils mit elektrischen Leiterbahnen 153 verbunden, die einerseits in einer Elektrodenstruktur 154 und andererseits in einer Kontaktstruktur 155 enden. Die Elektrodenstruktur 154 ragt jeweils in ein Testfeld 152 hinein und die Kontaktstruktur 155 dient zur Verbindung mit einer (nicht dargestellten) Messelektronik eines Analysegeräts. Der Grundkörper 151 mit den elektrischen Leiterbahnen 153, der Elektrodenstruktur 154 und der Kontaktstruktur 155 ist ein spritzgegossener Schaltungsträger (MID). Das Testelement 150 ist als runde Scheibe ausgebildet, auf der konzentrisch die Testfelder 152 mit zugehörigen Leiterbahnen 153 angeordnet sind. In einem Analysegerät kann dieses Testelement 150 automatisch oder manuell in eine Position gedreht werden, in der die elektrische Kontaktierung eines gewünschten Testfeldes 152 und die elektrochemische Analyse einer darauf vorhandenen Probe erfolgt.

Figur 14 zeigt eine zweite Ausführungsform eines im. Wesentlichen als spritzgegossener Schaltungsträger ausgeführten Testelements.

Das Testelement 160 weist einen Grundkörper 161 aus Kunststoff auf. In dem Grundkörper 161 sind Vertiefungen 162 ausgebildet, die eine zu analysierende Probe und ggf. ein Analysemittel aufnehmen können. Zur Analyse der Probe sind elektrische Leiterbahnen 163 vorgesehen die einerseits in einer Elektrodenstruktur 164 und andererseits in einer Kontaktstruktur 165 enden. Die Elektrodenstruktur 164 ragt jeweils in eine Vertiefung 162 hinein und die Kontaktstruktur 165 dient zur Verbindung mit einer (nicht dargestellten) Messelektronik eines Analysegeräts. Der Grundkörper 1.61 mit den elektrischen Leiterbahnen 163, der Elektrodenstruktur 164 und der Kontaktstruktur 165 ist ein spritzgegossener Schaltungsträger (MID). Das Testelement 160 ist als viereckiges Plättchen ausgebildet. Es enthält sechs Vertiefungen 162. Die elektrischen Leiterbahnen 163 sind so auf dem Testelement 160 angeordnet, dass alle Kontaktstrukturen 165 in einem begrenzten Bereich auf dem Testelement 160 positioniert sind. Dadurch vereinfacht sich die Positionierung des Testelements 160 zur elektrischen Kontaktierung der in verschiedenen Vertiefungen vorhandenen Proben in einem Analysegerät

### Bezugszeichenliste

- 1: Analysegerät
- 2: Gehäuse
- 3: Anzeigebereich
- 4: LCD
- 5: Bedienbereich
- 6: Einschalttaster
- 7: Testelementmagazinaufnahme
- 8: Testelementmagazin
- 9: Barcode
- 10: Kammer
- 11: Testelement
- 12: Stechhilfe
- 13: Testelementmagazinaufnahme-abdeckung
- 14: obere Gehäusehälfte
- 15: Leiterplatte
- 16: Motormodul
- 17: untere Gehäusehälfte
- 18: Typenschild
- 19: Batteriefachdeckel
- 20: Messmodul
- 21: Deckel
- 22: Entriegelungsknopf
- 23: Schubstangenmotormodul
- 24: Magazinmotormodul
- 25: Leiterplattenarm
- 26: Barcodereader
- 27: Antriebsrad
- 28: Motorhalter
- 29: Schubstange
- 30: Führungsbuchse
- 31: Führungseinrichtung
- 32: Motor
- 33: Positioniereinrichtung für ein Testelementmagazin
- 34: Segmentscheibe
- 35: Getriebeplatine
- 36: Barcodereaderanordnung
- 37: Gehäuseabschnitt
- 38: Testelementaufnahme
- 39: Optikplatine
- 40: Batteriekontakte
- 41: Magazinmotorkontakt
- 42: Schubstangenmotorkontakt
- 43: Messmodulkontakt
- 44: Positionsschalterkontakt
- 45: Schubstangenschalterkontakt
- 46: Schleifkontakt
- 47: Testelementkontakt
- 48: Optikplatinenkontakt
- 49: Barcodereaderkontakt
- 50: Motorkontakte
- 51: Magazinpositionskontakt
- 52: Verzahnung
- 53: Platine (MID)
- 54: Antriebsrad (MID)
- 55: Federkontakte
- 56: Segmentscheibe
- 57: Transporteinheit
- 58: Axialrichtung
- 59: Längsbohrung
- 60: Abstandhalter
- 61: Kontaktfahnen
- 62: Führungsbuchse (MID)
- 63: Führungseinrichtung
- 64: Längsbohrung
- 65: Kontaktfahnen
- 66: Richtung
- 67: Abstandhalter
- 68: Schaltelemente
- 69: Motormodul
- 70: Kontaktblech
- 71: federnde Kontaktfahnen
- 72: Motorhalter (MID)
- 73: Kontak-tfahnen
- 74: Leiterbahnen
- 75: weitere Bauteile
- 76: Barcodereaderanordnung
- 77: Gehäuseabschnitt (MID)
- 78: Leiterbahnen
- 79: federnde Kontakte
- 80: Veniegelungsschalter
- 81: Dornhülse
- 82: Abstützstelle
- 83: Riegel
- 84: Öffnungsrichtung
- 85: Schließrichtung
- 86: Sensor
- 87: Federkontakte
- 88: Leiterbahnen
- 89: Testelementmagazinaufnahme (MID)
- 90: Kontaktelement
- 91: Kunststoffteil
- 92: metallische Elemente
- 93: Auflagefläche
- 94: Kontaktrampen
- 95: Kontaktfahnen
- 96: Stecker
- 97: erstes Kontaktelement (MID)
- 98: Leiterbahnen
- 99: Kontaktrampen
- 100: Kunststoffteil
- 101: Kontaktfahne
- 102: Auflagefläche
- 103: zweites Kontaktelement (MID)
- 104: separate Kontaktfahnen
- 105: separate Kontaktrampen
- 106: Analysegerät Gehäusehälfte
- 107: elektrische Anschlüsse des Heizelements
- 108: keramisches Heizelement
- 109: Heizwendel
- 110: keramische Platte
- 111: Messmodul
- 112: Analysegerät Gehäusehälfte
- 113: Testelementaufnahme (MID)
- 114: Temperiereinrichtung
- 115: Heizwendel
- 116: federnder Kontakt
- 117: Vorratsbehältnis, Testelementmagazin
- 118: Kammern
- 119: Testelement
- 120: Barcode
- 121: elektrische Leiterbahnen
- 122: elektrische Kontakte
- 123: erstes elektrisch kontaktierendes Bauteil
- 124: zweites elektrisch kontaktierendes Bauteil
- 125: drittes elektrisch kontaktierendes Bauteil
- 126: viertes elektrisch kontaktierendes Bauteil
- 127: fünftes elektrisch kontaktierendes Bauteil
- 128: sechstes elektrisch kontaktierendes Bauteil
- 129: siebtes elektrisch kontaktierendes Bauteil
- 130: achtes elektrisch kontaktierendes Bauteil
- 131: neuntes elektrisch kontaktierendes Bauteil
- 132: zehntes elektrisch kontaktierendes Bauteil
- 150: Testelement
- 151: Grundkörper
- 152: Testfelder
- 153: Elektrische Leiterbahnenl
- 154: Elektrodenstruktur
- 155: Kontaktstruktur
- 160: Testelement
- 161: Grundkörper
- 162: Vertiefungen
- 163: Elektrische Leiterbahnenl
- 164: Elektrodenstruktur
- 165: Kontaktstruktur

## Patentansprüche

1. Analysegerät zur Analyse einer Probe auf einem Testelement, enthaltend mindestens ein elektrisch kontaktierendes Bauteil (123, 124, 125, 126, 127, 128, 129, 130, 131, 132), das zur Herstellung eines elektrischen Kontakts zu mindestens einem weiteren Bauteil zur Stromübertragung geeignet ist, **dadurch gekennzeichnet, dass** das elektrisch kontaktierende Bauteil (123, 124, 125, 126, 127, 128, 129, 130, 131, 132) ein spritzgegossener Schaltungsträger (MID) ist, wobei das elektrisch kontaktierende Bauteil (123, 124, 125, 126, 127, 128, 129, 130, 131, 132) mindestens eine federnde Kontaktfahne (55, 65, 73, 79, 87, 101, 104, 116) zur Kontaktierung des weiteren Bauteils aufweist, die einen Kontaktfahnenkörper aus spritzgegossenem Kunststoff und eine metallische Stromleiterstruktur enthält, wobei die Kontaktfahne (55, 65, 73, 79, 87, 101, 104, 116) ein rotierendes oder ein linear bewegtes weiteres Bauteil des Analysegeräts kontaktiert.

2. Analysegerät gemäß Anspruch 1, **dadurch gekennzeichnet, dass** das elektrisch kontaktierende Bauteil (123, 124, 125, 126, 127, 128, 129, 130, 131, 132) eine funktionale Baugruppe des Analysegeräts (1) und das weitere kontaktierte Bauteil eine Leiterplatte (15) des Analysegeräts (1) ist.

3. Analysegerät gemäß Anspruch 2, **dadurch gekennzeichnet, dass** die funktionale Baugruppe eine Barcodereaderanordnung (76), eine Transporteinheit (57) für Testelemente, ein Motormodul (69), ein Messmodul (20), eine Positioniereinrichtung (33) für ein Testelementmagazin (8), eine einen Sensor (86) enthaltende Testelementmagazinaufnahme (89) oder eine Temperiereinrichtung (114) ist.

4. Analysegerät gemäß Anspruch 1, **dadurch gekennzeichnet, dass** das elektrisch kontaktierende Bauteil ein Kontaktelement (97, 103) zur Testelementauswertung des Analysegeräts (1) und das kontaktierte weitere Bauteil ein elektrochemisch auszuwertendes Testelement (119) ist.

5. Analysegerät gemäß Anspruch 4, **dadurch gekennzeichnet, dass** das Kontaktelement zur Testelementauswertung in ein Testelementmagazin (117) integriert ist.

6. Analysegerät gemäß einem der Ansprüche 1 bis 5, wobei das Analysegerät (1) eine Positioniereinrichtung (33) für ein Testelementmagazin (8) enthält, die eine Platine (53) zur Stromversorgung und ein durch einen Motor angetriebenes Antriebsrad (54) zum Antreiben des drehbaren Testelementmagazins (8) umfasst, **dadurch gekennzeichnet, dass** die Platine (53) mit Federkontakten (55) als spritzgegossener Schaltungsträger und das Antriebsrad (54) mit einer elektrisch kontaktierbaren Segmentscheibe (56) als spritzgegossener Schaltungsträger ausgeführt ist

7. Analysegerät gemäß einem der Ansprüche 1 bis 6, wobei das Analysegerät (1) eine Transporteinheit (57) für Testelemente enthält, die eine Schubstange (29) zum Transport eines Testelements in dem Analysegerät (1) umfasst, wobei die Schubstange (29) eine Führungsbuchse (62) aufweist, die in einer Führungseinrichtung (63) beim Transport des Testelements führbar ist, **dadurch gekennzeichnet, dass** die Führungsbuchse (62) federnde Kontaktfahnen (65) zum Kontaktieren einer Leiterplatte aufweist und die Führungseinrichtung (63) Schaltelemente (68) umfasst, die so angeordnet sind, dass sie die Kontaktfahnen (65) in bestimmten Positionen der Führungsbuchse (62) in der Führungseinrichtung (63) gegen die Leiterplatte drücken, wobei die Führungsbuchse (62) mit den Kontaktfahnen (65) ein spritzgegossener Schaltungsträger ist.

8. Analysegerät gemäß einem der Ansprüche 1 bis 7, wobei das Analysegerät (1) eine Barcodereaderanordnung (76) enthält, die einen Gehäuseabschnitt (77) des Analysegeräts (1) umfasst, in dem ein Barcodereader (26) angeordnet ist, **dadurch gekennzeichnet, dass** der Gehäuseabschnitt (77) zu dem Barcodereader (26) verlaufende Leiterbahnen (78) und federnde Kontakte (79) zum Kontaktieren einer Leiterplatte enthält und der Gehäuseabschnitt (77) mit den Leiterbahnen (78) und federnden Kontakten (79) ein spritzgegossener Schaltungsträger ist.

9. Analysegerät gemäß einem der Ansprüche 1 bis 8, wobei das Analysegerät (1) eine einen Sensor (86) enthaltende Testelementmagazinaufnahme (89) umfasst, die zu dem Sensor (86) verlaufende elektrische Leiterbahnen (88) enthält, wobei die Testelementmagazinaufnahme (89) mit den Leiterbahnen (88) ein spritzgegossener Schaltungsträger ist.

10. Analysegerät gemäß einem der Ansprüche 1 bis 9, wobei das Analysegerät (1) ein Motormodul (69) enthält, das einen Motor (32) und einen Motorhalter (72) umfasst, wobei der Motorhalter (72) Kontaktfahnen (73) zum Kontaktieren einer Leiterplatte aufweist und der Motorhalter (72) mit den Kontaktfahnen (71) ein spritzgegossener Schaltungsträger ist.

11. Analysegerät gemäß einem der Ansprüche 1 bis 10, wobei das Analysegerät (1) ein Messmodul (111) zum Durchführen einer Messung an einem auf einem Testelement enthaltenen Analyten enthält, das eine Testelementaufnahme (113) zum Aufnehmen des Testelements während der Messung umfasst, in der eine Temperiereinrichtung (114) zum Temperieren des Testelements enthalten ist, **dadurch gekennzeichnet, dass** die Testelementaufnahme (113) mit der Temperiereinrichtung (114), die Heizwendeln (115) und einen federnden Kontakt (116) umfasst, ein spritzgegossener Schaltungsträger ist.

12. Analysegerät gemäß einem der Ansprüche 1 bis 11, wobei das Analysegerät (1) ein Kontaktelement (97, 103) zur Testelementauswertung enthält, das eine Auflagefläche (102) für ein Testelement aufweist, wobei über die Auflagefläche (102) eine Vielzahl von Kontaktrampen (99, 105) hinausragen, die dazu vorgesehen sind, um elektrische Kontakte zu einem auf der Auflagefläche (102) positionierten Testelement herzustellen und die über auf dem Kontaktelement (97, 103) verlaufenden Leiterbahnen (98) mit einer Leiterplatte verbindbar sind, wobei das Kontaktelement (97, 103) ein spritzgegossener Schaltungsträger ist.

13. Verfahren zur Herstellung eines Analysegeräts zur Analyse einer Probe auf einem Testelement, enthaltend mindestens ein elektrisch kontaktierendes Bauteil, das zur Herstellung eines elektrischen Kontakts zu mindestens einem weiteren Bauteil zur Stromübertragung geeignet ist, wobei das elektrisch kontaktierende Bauteil (123, 124, 125, 126, 127, 128, 129, 130, 131, 132) mindestens eine federnde Kontaktfahne (55, 65, 73, 79, 87, 101, 104, 116) zur Kontaktierung des weiteren Bauteils aufweist, die einen Kontaktfahnenkörper aus spritzgegossenem Kunststoff und eine metallische Stromleiterstruktur enthält, wobei die Kontaktfahne (55, 65, 73, 79, 87, 101, 104, 116) ein rotierendes oder ein linear bewegtes weiteres Bauteil des Analysegeräts kontaktiert, wobei das Verfahren folgende Schritte aufweist:
A) Herstellen des elektrisch kontaktierenden Bauteils, das einen Grundkörper und eine metallische Leiterstruktur umfasst, durch ein Verfahren zur Herstellung spritzgegossener Schaltungsträger (MID) und
B) Positionieren und Montieren des elektrisch kontaktierenden Bauteils in dem Analysegerät.

14. Verfahren gemäß Anspruch 13, **dadurch gekennzeichnet, dass** das Verfahren zur Herstellung spritzgegossener Schaltungsträger (MID) ein Verfahren ausgewählt aus der Gruppe Zweikomponentenspritzgießen, Heißprägen, Folienhinterspritzen und Laserstrukturierung ist.

## Claims

1. Analysis apparatus for analysing a sample on a test element, containing at least one electrically contacting structural part (123, 124, 125, 126, 127, 128, 129, 130, 131, 132), which is suitable for forming an electrical contact with at least one further structural part for the transmission of current, **characterised in that** the electrically contacting structural part (123, 124, 125, 126, 127, 128, 129, 130, 131, 132) is an injection moulded circuit carrier (MID), wherein the electrically contacting structural part (123, 124, 125, 126, 127, 128, 129, 130, 131, 132) comprises at least one elastically resilient contact tag (55, 65, 73, 79, 87, 101, 104, 116) for contacting the said further structural part, which contains a contact tag body of injection moulded plastic and a metallic current conductor structure, wherein the contact tag (55, 65, 73, 79, 87, 101, 104, 116) contacts a rotating or a linearly moving further structural part of the analysis apparatus,

2. Analysis apparatus according to claim 1, **characterised in that** the electrically contacting structural part (123, 124, 125, 126, 127, 128, 129, 130, 131, 132) is a functional component of the analysis apparatus (1) and the further contacted structural part is a printed circuit board (15) of the analysis apparatus (1).

3. Analysis apparatus according to claim 2, **characterised in that** the functional component is a bar code arrangement (76), a transporting unit (57) for test elements, a motor module (69), a measuring module (20), a positioning device (33) for a test element cartridge (8), a test element cartridge receptacle (89) containing a sensor (86), or a temperature control device (114).

4. Analysis apparatus according to claim 1, **characterised in that** the electrically contacting structural part is a contact element (97, 103) for the test element evaluation of the analysis apparatus (1), and the contacted further structural part is a test element (119) to be evaluated electrochemically.

5. Analysis apparatus according to claim 4, **characterised in that** the contact element for the test element evaluation is integrated in a test element cartridge (117) .

6. Analysis apparatus according to one of claims 1 to 5, wherein the analysis apparatus (1) contains a positioning device (33) for a test element cartridge (8), which includes a plate (53) for the current supply and a drive wheel (54) driven by a motor for driving the rotatable test element cartridge (8), **characterised in that** the plate (53) is designed with spring contacts (55) as an injection moulded circuit carrier, and the drive wheel (54) is designed with an electrically contactable segment disc (56) as an injection moulded circuit carrier.

7. Analysis apparatus according to one of claims 1 to 6, wherein the analysis apparatus (1) contains a transporting unit (57) for test elements, which includes a connecting rod (29) for transporting a test element in the analysis apparatus (1), wherein the connecting rod (29) comprises a guide bushing (62) that can be guided in a guide device (63) during transportation of the test element, **characterised in that** the guide bushing (62) comprises elastically resilient contact tags (65) for contacting a printed circuit board and the guide device (63) comprises switch elements (68) that are arranged so that the contact tags (65) in specific positions of the guide bushing (62) in the guide device (63) press against the printed circuit board, wherein the guide bushing (62) with the contact tags (65) is an injection moulded circuit carrier.

8. Analysis apparatus according to one of claims 1 to 7, wherein the analysis apparatus (1) contains a bar code arrangement (76), which includes a housing section (77) of the analysis apparatus (1) in which a bar code reader (26) is arranged, **characterised in that** the housing section (77) contains conducting tracks (78) running to the bar code reader (26) and elastically resilient contacts (79) for contacting a printed circuit board, and the housing section (77) with the conducting tracks (78) and resiliently elastic contacts (79) is an injection moulded circuit carrier.

9. Analysis apparatus according to one of claims 1 to 8, wherein the analysis apparatus (1) comprises a test element cartridge receptacle (89) containing a sensor (86), which contains electrical conducting strips (88) running to the sensor (86), wherein the test element cartridge receptacle (89) with the conducting tracks (88) is an injection moulded circuit carrier.

10. Analysis apparatus according to one of claims 1 to 9, wherein the analysis apparatus (1) contains a motor module (69), which includes a motor (32) and a motor holder (72), wherein the motor holder (72) comprises contact tags (73) for contacting a printed circuit board and the motor holder (72) with the contact tags (71) is an injection moulded circuit carrier.

11. Analysis apparatus according to one of claims 1 to 10, wherein the analysis apparatus (1) is a measurement module (111) for carrying out a measurement on an analyte contained on a test element, which comprises a test element receptacle (113) for receiving the test element during the measurement, in which a temperature control device (114) is contained for controlling the temperature of the test element, **characterised in that** the test element receptacle (113) with the temperature control device (114), which includes heating coils (115) and a resiliently elastic contact (116), is an injection moulded circuit carrier.

12. Analysis apparatus according to one of claims 1 to 11, wherein the analysis apparatus (1) contains a contact element (97, 103) for the test element evaluation, which comprises a support surface (102) for a test element, wherein a plurality of contact ramps (99, 105) project over the support surface (102), which are provided so as to make electrical contact with a test element positioned on the support surface (102) and which can be connected to a printed circuit board via conducting tracks (98) running on the contact element (97, 103), wherein the contact element (97, 103) is an injection moulded circuit carrier.

13. Method for producing an analysis apparatus for analysing a sample on a test element, containing at least one electrically contacting structural part that is suitable for forming an electrical contact with at least one further structural part for the transmission of current, wherein the electrically contacting structural part (123, 124, 125, 126, 127, 128, 129, 130, 131, 132) comprises at least one resiliently elastic contact tag (55, 65, 73, 79, 87, 101, 104, 116) for contacting the said further structural part, which contains a contact tag body of injection moulded plastic and a metallic current conducting structure, wherein the contact tags (55, 65, 73, 79, 87, 101, 104, 116) contact a rotating or a linearly moving further structural part of the analysis apparatus, wherein the method comprises the following steps:
A) production of the electrically contacting structural part, which comprises a base body and a metallic conducting structure, by a method for producing injection moulded circuit carriers (MID), and
B) positioning and installation of the electrically contacting structural part in the analysis apparatus.

14. Method according to claim 13, **characterised in that** the method for producing injection moulded circuit carriers (MID) is a method selected from the group comprising two-component injection moulding, hot embossing, film back-injection and laser structuring.

## Revendications

1. Appareil d'analyse pour l'analyse d'un échantillon sur un élément d'essai, contenant au moins un composant établissant un contact électrique (123, 124, 125, 126, 127, 128, 129, 130, 131, 132) qui est approprié pour l'établissement d'un contact électrique avec au moins un composant supplémentaire pour la transmission du courant, **caractérisé en ce que** le composant établissant un contact électrique (123, 124, 125, 126, 127, 128, 129, 130, 131, 132) est un support de circuits moulé par injection (MID), le composant établissant un contact électrique (123, 124, 125, 126, 127, 128, 129, 130, 131, 132) présentant au moins une langue de contact élastique (55, 65, 73, 79, 87, 101, 104, 116) pour la mise en contact avec le composant supplémentaire, qui contient un corps de langue de contact constitué d'une matière synthétique moulée par injection et une structure métallique électroconductrice, la langue de contact (55, 65, 73, 79, 87, 101, 104, 116) entrant en contact avec un composant supplémentaire de l'appareil de mesure, de type rotatif ou à déplacement linéaire.

2. Appareil d'analyse selon la revendication 1, **caractérisé en ce que** le composant établissant un contact électrique (123, 124, 125, 126, 127, 128, 129, 130, 131, 132) est un module fonctionnel de l'appareil d'analyse (1) et le composant supplémentaire mis en contact est une plaquette de circuits imprimés (15) de l'appareil d'analyse (1).

3. Appareil d'analyse selon la revendication 2, **caractérisé en ce que** le module fonctionnel est un agencement de lecture de codes-barres (76), une unité de transport (57) pour des éléments d'essai, un module faisant office de moteur (69), un module de mesure (20), un mécanisme de positionnement (33) pour un magasin d'éléments d'essai (8), un logement de magasin d'éléments d'essai (89) contenant un capteur (86) ou un mécanisme de maintien de la température (114).

4. Appareil d'analyse selon la revendication 1, **caractérisé en ce que** le composant établissant un contact électrique est un élément de contact (97, 103) pour l'évaluation d'un élément d'essai de l'appareil d'analyse (1) et le composant supplémentaire mis en contact est un élément d'essai (119) à évaluer par voie électrochimique.

5. Appareil d'analyse selon la revendication 4, **caractérisé en ce que** l'élément de contact pour l'évaluation d'un élément d'essai est intégré dans un magasin d'éléments d'essai (117).

6. Appareil d'analyse selon l'une quelconque des revendications 1 à 5, dans lequel l'appareil d'analyse (1) contient un mécanisme de positionnement (33) pour un magasin d'éléments d'essai (8) qui comprend une platine (53) pour l'alimentation en courant et une roue d'entraînement (54) entraînée par un moteur, pour l'entraînement du magasin d'éléments d'essai (8) de type rotatif, **caractérisé en ce que** la platine (53) est réalisée avec des contacts élastiques (55) sous la forme d'un support de circuits moulé par injection et la roue d'entraînement (54) est réalisée avec un disque segmenté (56) apte à une mise en contact électrique sous la forme d'un support de circuits moulé par injection.

7. Appareil d'analyse selon l'une quelconque des revendications 1 à 6, dans lequel l'appareil d'analyse contient une unité de transport (57) pour des éléments d'essai, qui comprend une barre de poussée (29) pour le transport d'un élément d'essai dans l'appareil d'analyse (1), la barre de poussée (29) présentant une douille de guidage (62) qui peut être guidée dans un dispositif de guidage (63) lors du transport de l'élément d'essai, **caractérisé en ce que** la douille de guidage (62) présente des langues de contact élastiques (65) pour la mise en contact avec une plaquette de circuits imprimés et le mécanisme de guidage (63) comprend des éléments de circuits (68) qui sont disposés de telle sorte qu'ils poussent les langues de contact (65), dans des positions déterminées de la douille de guidage (62) dans le mécanisme de guidage (63), contre la plaquette de circuits imprimés, la douille de guidage (62) représentant, avec les langues de contact (65), un support de circuits moulé par injection.

8. Appareil d'analyse selon l'une quelconque des revendications 1 à 7, dans lequel l'appareil d'analyse (1) contient un agencement de lecture de codes-barres (75) qui comprend une section de logement (77) de l'appareil d'analyse (1) dans laquelle est disposé un lecteur de codes-barres (26), **caractérisé en ce que** la section de logement (77) contient des pistes conductrices (78) s'étendant en direction du lecteur de codes-barres (26) et des contacts élastiques (79) pour la mise en contact avec une plaquette de circuits imprimés, et la section de logement (77) représente, avec les pistes conductrices (78) et les contacts élastiques (79), un support de circuits moulé par injection.

9. Appareil d'analyse selon l'une quelconque des revendications 1 à 8, dans lequel l'appareil d'analyse (1) comprend un logement de magasin d'éléments d'essai (89) contenant un capteur (86), qui contient des pistes électroconductrices (88) s'étendant en direction du capteur (86), le logement de magasin d'éléments d'essai (89) représentant, avec les pistes conductrices (88), un support de circuits moulé par injection.

10. Appareil d'analyse selon l'une quelconque des revendications 1 à 9, dans lequel l'appareil d'analyse (1) contient un module (69) faisant office de moteur, qui comprend un moteur (32) et un support de moteur (72), le support de moteur (72) présentant des langues de contact (73) pour la mise en contact avec une plaquette de circuits imprimés, et le support de moteur (72) représentant, avec les langues de contact (71), un support de circuits moulé par injection.

11. Appareil d'analyse selon l'une quelconque des revendications 1 à 10, dans lequel l'appareil d'analyse (1) contient un module de mesure (111) pour effectuer une mesure d'un analyte contenu sur un élément d'essai, qui comprend un logement d'élément d'essai (113) pour la réception de l'élément d'essai au cours de la mesure, dans lequel est contenu un mécanisme de maintien de la température (114) pour le maintien de la température de l'élément d'essai, **caractérisé en ce que** le logement d'élément d'essai (113), qui comprend le dispositif de maintien de la température (114), les serpentins de chauffage (115) et un contact élastique (116), représente un support de circuits moulé par injection.

12. Appareil d'analyse selon l'une quelconque des revendications 1 à 11, dans lequel l'appareil d'analyse (1) contient un élément de contact (97, 103) pour évaluer l'élément d'essai qui présente une surface d'appui (102) pour un élément d'essai, une multitude de rampes de contact (99, 105) faisant saillie au-delà de la surface d'appui (102), qui sont prévues pour établir des contacts électriques avec un élément d'essai disposé sur la surface d'appui (102) et qui peuvent être reliées avec une plaquette de circuits imprimés via les pistes conductrices (198) s'étendant sur l'élément de contact (97, 103), l'élément de contact (97, 103) représentant un support de circuits moulé par injection.

13. Procédé pour la fabrication d'un appareil d'analyse destiné à l'analyse d'un échantillon sur un élément d'essai, contenant au moins un composant établissant un contact électrique qui est approprié pour l'établissement d'un contact électrique avec au moins un composant supplémentaire pour la transmission du courant, le composant établissant un contact électrique (123, 124, 125, 126, 127, 128, 129, 130, 131, 132) présentant au moins une langue de contact élastique (55, 65, 73, 79, 87, 101, 104, 116) pour la mise en contact avec le composant supplémentaire, qui contient un corps de langue de contact constitué d'une matière synthétique moulée par injection et une structure métallique électroconductrice, la langue de contact (55, 65, 73, 79, 87, 101, 104, 116) entrant en contact avec un composant supplémentaire de l'appareil de mesure, de type rotatif ou à déplacement linéaire, le procédé présentant les étapes suivantes:
A) la fabrication d'un composant établissant un contact électrique, qui comprend un corps de base et une structure conductrice métallique, via un procédé pour la fabrication de supports de circuits moulés par injection (MID) et
B) le positionnement et le montage du composant établissant un contact électrique dans l'appareil d'analyse.

14. Procédé selon la revendication 13, **caractérisé en ce que** le procédé pour la fabrication de supports de circuits moulés par injection (MID) est un procédé choisi parmi le groupe du moulage par injection à deux composants, de l'estampage à chaud, du moulage à film et de la structuration au laser.
